(19) Europäisches Patentamt
European Patent Office
Office européen des brevets

(11) EP 1 342 464 B1

(12) **FASCICULE DE BREVET EUROPEEN**

(45) Date de publication et mention de la délivrance du brevet:
**11.10.2006 Bulletin 2006/41**

(51) Int Cl.:
***A61K 8/81*** *(2006.01)* ***A61K 8/89*** *(2006.01)*
***A61K 8/92*** *(2006.01)* ***A61Q 1/06*** *(2006.01)*

(21) Numéro de dépôt: **02293089.5**

(22) Date de dépôt: **13.12.2002**

(54) **Composition de maquillage ou de soin des matières kératiniques comprenant un composé siliconé non volatil, un polymère liposoluble non siliconé et un agent dispersant particulier**

Zusammensetzung zum Schminken oder zum Pflegen von Keratinstoffe enthaltend eine nichtflüchtige Silikonverbindung, ein silikonfreies fettlösliches Polymer sowie ein bestimmtes Dispergiermaterial

Composition for make-up or care of keratinic materials comprising a non-volatile silicone component, a non-silicone liposoluble polymer and a specific dispersing agent

(84) Etats contractants désignés:
**AT BE BG CH CY CZ DE DK EE ES FI FR GB GR IE IT LI LU MC NL PT SE SI SK TR**

(30) Priorité: **08.01.2002 FR 0200160**

(43) Date de publication de la demande:
**10.09.2003 Bulletin 2003/37**

(73) Titulaire: **L'ORÉAL**
**75008 Paris (FR)**

(72) Inventeurs:
- **Arnaud, Pascal**
  **94240 L'Hay les Roses (FR)**
- **Filippi, Vanina**
  **75015 Paris (FR)**
- **Blin, Xavier**
  **75015 Paris (FR)**

(74) Mandataire: **Le Coupanec, Pascale A.M.P.**
**Nony & Associés**
**3, rue de Penthièvre**
**75008 Paris (FR)**

(56) Documents cités:
**EP-A- 1 112 734** **EP-A- 1 184 028**
**WO-A-97/16157**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

**Description**

La présente invention se rapporte à une composition cosmétique anhydre contenant un agent dispersant particulier. Cette composition possède des propriétés cosmétiques remarquables, en particulier de tenue, et confère au maquillage ou au soin des propriétés de brillance, de confort et de non-migration.

Cette composition peut se présenter notamment sous forme de produit coulé en stick ou en coupelle comme les rouges ou baumes à lèvres, les fonds de teint coulés, les produits anti-cernes, les fards à paupières ou à joues, sous forme de pâte ou de crème plus ou moins fluide comme les fonds de teint ou rouges à lèvres fluides, les eye liners, les mascaras, les compositions de protection solaire, de coloration ou de bronzage artificiel de la peau ou encore de maquillage du corps ou des cheveux.

Les produits de maquillage ou de soin de la peau ou des lèvres des êtres humains comme les fonds de teint ou les rouges à lèvres contiennent généralement des phases grasses telles que des cires et des huiles, des pigments et/ou charges et éventuellement des additifs comme des actifs cosmétiques. Elles peuvent aussi contenir des produits dits "pâteux", de consistance souple, permettant d'obtenir des pâtes, colorées ou non, à appliquer au pinceau.

Les compositions, notamment de maquillage, connues ont tendance à migrer, c'est-à-dire à se propager au cours du temps à l'intérieur des plis des rides et des ridules de la peau qui entourent notamment les lèvres et les yeux, entraînant un effet inesthétique. Cette migration est souvent citée par les femmes comme un défaut majeur des rouges à lèvres et des fards à paupières classiques. Par "migration", on entend un débordement de la composition et en particulier de la couleur, hors du tracé initial du maquillage.

En outre, ces compositions présentent une mauvaise tenue dans le temps et en particulier de la couleur. Cette mauvaise tenue se caractérise par une modification de la couleur (virage, palissement) généralement par suite d'une interaction avec le sébum et/ou la sueur sécrétés par la peau dans le cas de fond de teint et de fard à joues ou à paupières, ou d'une interaction avec la salive dans le cas des rouges à lèvres. De plus, cette modification de couleur est souvent non homogène. Ceci oblige l'utilisateur à se remaquiller très souvent, ce qui peut constituer une perte de temps.

Les problèmes de tenue de la couleur étaient jusqu'ici résolus en introduisant un taux élevé de pigments dans les compositions, de manière à ce que celles-ci déposent, sur le support sur lequel elles sont appliquées, une quantité importante de pigments.

Il a également été proposé des compositions contenant des composés volatils, qui, bien que présentant des propriétés de tenue améliorées, ont l'inconvénient de laisser sur la peau et les lèvres, après évaporation des composés volatils, un film qui devient inconfortable au cours du temps (sensation de dessèchement et de tiraillement), écartant un certain nombre de femmes de ce type de rouge à lèvres. En outre, ces compositions conduisent à des films colorés mats et couvrants. Or, les femmes sont aujourd'hui à la recherche de produits, notamment de coloration des lèvres ou des paupières, brillants et semi-couvrants.

Il est connu par ailleurs que l'amélioration des propriétés de brillance nécessite une bonne dispersion des particules solides dans la composition, en particulier des pigments.

Le brevet US-A-5 945 092 de Revlon décrit ainsi l'utilisation de tensioactifs siliconés associés avec des huiles volatiles et des agents dispersants fluorés. Cependant, comme exposé plus haut, l'utilisation de composés volatils entraîne certains inconvénients. De plus, malgré leur efficacité, ces tensioactifs présentent l'inconvénient d'être potentiellement irritant, notamment pour la muqueuse labiale, lorsque leur pourcentage dans la composition est important (typiquement supérieur à 3 %), et ceci d'autant plus que le taux d'huile volatile est élevé (supérieur à 30 % typiquement). Les agents dispersants fluorés décrits dans les exemples de ce brevet n'ont pas les paramètres de solubilité tels que définis selon la présente invention.

La société Kao a proposé dans sa demande EP-A-0548694 une composition contenant un tensioactif siliconé (silicone modifiée polyéther), des huiles et des pigments, ayant un bon confort à l'utilisation et une tenue améliorée. Toutefois, ces compositions ne permettent pas d'obtenir un maquillage ayant une tenue suffisante.
De plus, on privilégie actuellement, dans le domaine de la cosmétique, l'utilisation de composés d'origine naturelle. Or les tensio actifs siliconés et fluorés prévus dans les compositions des documents ci-dessus sont d'origine synthétique.

La demande EP 1 112 734 (L'OREAL) décrit des compositions contenant un tensio-actif hydrocarboné, un composé siliconé non volatile et un polymère liposoluble (sous la forme d'un pâteux). Cependant, les tensio-actifs hydrocarbonés donnés en exemple (comme le tri-isostéarate de diglyoéryle ou l'acide poly (hydroxy-12) stéarique) ont un paramètre de solubilité a compris entre 2 et 9,08 $(J/cm^3)^{1/2}$. L'exemple 2 comparatif de la présente demande montre qu'une composition contenant un agent dispersant tel que le paramètre $\delta_a$ est compris entre 9,1 et 20 permet d'améliorer la tenue du maquillage tout en gardant la même brillance.

Il subsiste donc le besoin d'une composition ne présentant pas les inconvénients ci-dessus et ayant notamment de bonnes propriétés de tenue, qui migre peu ou pas, tout en conférant au dépôt de maquillage ou de soin un aspect plus ou moins brillant, adapté au désir de la consommatrice, ne desséchant pas et ne tiraillant pas la peau ou les lèvres sur lesquelles elle est appliquée, aussi bien lors de l'application qu'au cours du temps, et n'irritant pas la peau ou les lèvres.

Le demandeur a constaté, de façon surprenante, que l'utilisation de l'association d'au moins un composé siliconé non volatil, d'au moins un polymère liposoluble non siliconé et d'au moins un agent dispersant hydrocarboné permettait l'obtention d'une composition de bonne tenue, notamment en couleur, brillante, confortable et qui migre peu ou pas, et non irritante.

La présente invention a donc pour objet une composition anhydre de soin ou de maquillage des matières kératiniques, comprenant un milieu cosmétiquement acceptable contenant au moins un composé siliconé non volatil, au moins un polymère liposoluble non siliconé et au moins un agent dispersant hydrocarboné qui présente les paramètres de solubilité δd et δa satisfaisant aux conditions suivantes : $16,2 \leq \delta_d \leq 20$ $(J/cm^3)^{1/2}$ et $9,1 \leq \delta_a \leq 20$ $(J/cm^3)^{1/2}$, ledit agent dispersant comportant des atomes de carbone et d'hydrogène et une ou plusieurs fonctions choisies parmi les fonctions hydroxyle, ester, éther, carboxylique ou amide.

Par « au moins » un composé, on entend un ou plusieurs composés.

Par « composition cosmétique anhydre », on entend une composition comprenant une phase continue (appelée aussi phase externe) grasse qui représente jusqu'à 95 % en poids de la composition, de préférence jusqu'à 98% en poids, de préférence encore jusqu'à 99,5 % en poids.

L'invention a également pour objet un procédé cosmétique pour conférer à un film de composition cosmétique anhydre des propriétés de tenue de la couleur, de brillance, de confort et/ou de non-migration, consistant à introduire dans ladite composition au moins un composé siliconé non volatil, au moins un polymère liposoluble non siliconé et au moins un agent dispersant hydrocarboné qui présente les paramètres de solubilité δd et δa satisfaisant aux conditions suivantes : $16,2 \leq \delta_d \leq 20$ $(J/cm^3)^{1/2}$ et $9,1 \leq \delta_a \leq 20$ $(J/cm^3)^{1/2}$.

L'invention a encore pour objet l'utilisation de l'association d'au moins un composé siliconé non volatil, d'au moins un polymère liposoluble non siliconé et d'au moins un agent dispersant hydrocarboné qui présente les paramètres de solubilité δd et δa satisfaisant aux conditions suivantes : $16,2 \leq \delta_d \leq 20$ $(J/cm^3)^{1/2}$ et $9,1 \leq \delta_a \leq 20$ $(J/cm^3)^{1/2}$, dans une composition cosmétique anhydre de bonne tenue, notamment de la couleur, brillante, confortable et/ou non migrante.

L'invention a enfin pour objet l'utilisation de l'association d'au moins un composé siliconé non volatil, d'au moins un polymère liposoluble non siliconé et d'au moins un agent dispersant hydrocarboné qui présente les paramètres de solubilité δd et δa satisfaisant aux conditions suivantes : $16,2 \leq \delta_d \leq 20$ $(J/cm^3)^{1/2}$ et $9,1 \leq \delta_a \leq 20$ $(J/cm^3)^{1/2}$, dans une composition cosmétique anhydre, comme agent pour conférer à ladite composition des propriétés de tenue, notamment de la couleur, de brillance, de confort et/ou de non-migration.

Le composé siliconé non volatil, le polymère liposoluble non siliconé et l'agent dispersant hydrocarboné de la composition selon l'invention sont des composés distincts.

Par « matières kératiniques », on entend la peau, les lèvres et les phanères.

Par composé "non-volatil", on entend un composé susceptible de rester sur la peau ou les lèvres pendant plusieurs heures. Un composé non-volatil a en particulier une pression de vapeur, à température ambiante et pression atmosphérique, non nulle, inférieure à 0,02 mm de Hg (2,66 Pa). Par composé "volatil", on entend un composé susceptible de s'évaporer de la peau ou des lèvres, en moins d'une heure. Un composé volatil est notamment choisi parmi les composés ayant une pression de vapeur, à température ambiante et pression atmosphérique allant de 0,02 mm à 300mm de Hg (2,66 Pa à 40 000 Pa) et mieux allant de 0,1 à 90 mm de Hg (13 Pa à 12 000 Pa).

Par polymère « liposoluble », on entend un polymère non cireux, liquide ou pâteux à température ambiante (25°C), soluble dans les huiles selon l'invention.

La composition selon l'invention contient donc des ingrédients compatibles avec les matières kératiniques, à savoir la peau, les lèvres, les fibres kératiniques et les ongles. Elle peut se présenter sous forme plus ou moins fluide, de pâte ou de solide non déformable ou rigide, éventuellement coulé en stick ou en coupelle. De préférence, elle se présente sous forme fluide ou de stick en particulier anhydre. Par "fluide", on entend une composition s'écoulant sous son propre poids, à l'inverse d'un solide.

La composition de l'invention contient peu ou pas d'huiles volatiles c'est à dire moins de 10 % par rapport au poids total de la composition, de préférence moins de 5 % et mieux moins de 2 % et avantageusement est exempte d'huile volatile.

L'agent dispersant hydrocarboné utilisé dans la composition selon l'invention sert à protéger les particules dispersées contre leur agglomération ou floculation. Par composé « hydrocarboné », on entend un composé comportant des atomes de carbone et d'hydrogène et une ou plusieurs fonctions choisies parmi les fonctions hydroxyle, ester, éther (oxyde d'éthylène), carboxylique, amide.

L'agent dispersant hydrocarboné de l'invention est dépourvu d'atomes de fluor. Cet agent porte une ou des fonctionnalités ayant une affinité forte pour la surface des particules à disperser.

De préférence, l'agent dispersant hydrocarboné (appelé par la suite «dispersant hydrocarboné»), de la composition selon l'invention, est fluide à température ambiante (25°C), et notamment liquide et/ou présente un indice de réfraction ≥ 1,45 à 20°C (l'indice de réfraction étant mesuré au réfractomètre).

Ce dispersant hydrocarboné présente les paramètres de solubilité δd et δa selon l'espace de solubilité de Hansen satisfaisant aux conditions suivantes :

$16{,}2 \leq \delta_d \leq 20$ $(J/cm^3)^{1/2}$ de préférence $16{,}3 \leq \delta_d \leq 19$ $(J/cm^3)^{1/2}$, et mieux $16{,}9 \leq \delta_d \leq 18$ $(J/cm^3)^{1/2}$
et
$9{,}1 \leq \delta_a \leq 20$ $(J/cm^3)^{1/2}$, de préférence $10 \leq \delta_a \leq 18{,}1$ $(J/cm^3)^{1/2}$, et mieux $13 \leq \delta_a \leq 14{,}5$ $(J/cm^3)^{1/2}$

La définition des paramètres de solubilité selon HANSEN est bien connue de l'homme du métier, et notamment décrite dans l'article de C. M. HANSEN : "The three dimensional solubility parameters" J.

Paint Technol. 39, 105 (1967). Ces paramètres sont aussi décrits dans le document JP-A-08-109121 de KAO et le document de D.W. Van KREVELEN "Properties of polymers" (1990), p. 190.

Selon cet espace de HANSEN :

- $\delta_d$ caractérise les forces de dispersion de LONDON issues de la formation de dipôles induits lors des chocs moléculaires ;
- $\delta_p$ caractérise les forces d'interactions de DEBYE entre dipôles permanents ;
- $\delta_h$ caractérise les forces d'interactions spécifiques (type liaisons hydrogène, acide/base, donneur/accepteur, etc.) ;

Les paramètres $\delta_d$, $\delta_p$ et $\delta_h$ sont généralement exprimés en $(J/cm^3)^{1/2}$. Ils sont déterminés à température ambiante (25°C) et en particulier selon la méthode de calcul indiquée dans le document brevet de KAO ci-dessus.

Dans la composition selon l'invention, on peut utiliser n'importe quel dispersant hydrocarboné fluide, et en particulier liquide, ou mélange de dispersants hydrocarbonés fluides satisfaisant aux relations ci-dessus. Dans ce cas, les paramètres de solubilité du mélange sont déterminés à partir de ceux des dispersants hydrocarbonés fluides pris séparément, selon les relations suivants :

$$\delta_{Dmel} = \sum_i xi\, \delta_{Di} \;; \qquad \delta_{pmel} = \sum_i xi\, \delta_{pi} \quad \text{et} \quad \delta_{hmel} = \sum_i xi\, \delta_{hi}$$

où xi représente la fraction volumique du dispersant hydrocarboné fluide (i) dans le mélange.

Il est à la portée de l'homme du métier de déterminer les quantités de chaque dispersant hydrocarboné fluide pour obtenir un mélange de dispersants hydrocarbonés fluides satisfaisant aux relations ci-dessus.

Avantageusement, le dispersant hydrocarboné présente une structure chimique comportant au moins un groupement polaire choisi parmi -COOH ; -OH ; oxyde d'éthylène :

- $(O\text{-}CH_2\text{-}CH_2\text{-})$ ; oxyde de propylène

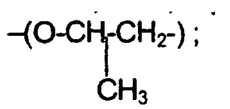

- $PO_4$ ; NHR ; $NR_1R_2$ avec $R_1$, $R_2$ formant éventuellement un cycle et représentant un radical alkyle ou alkoxy linéaire ou ramifié en $C_1$ à $C_{20}$ ou

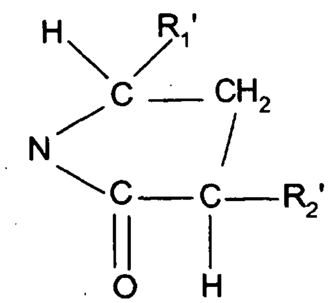

avec $R_1$' et $R_2$' qui peuvent être égal à H ou à une chaîne alkyle ou alkoxy linéaire ou ramifiée en $C_1$ à $C_{20}$.

Le dispersant hydrocarboné selon l'invention peut être choisi parmi :

- les alcools gras modifiés éther et en particulier les produits d'addition de l'oxyde d'éthylène et /

ou de l'oxyde de propylène avec i) un alcool gras linéaire ou ramifié ou avec ii) un alkylphénol,

- les esters résultant de la réaction d'au moins un acide gras avec au moins un produit d'addition de l'oxyde d'éthylène et du glycérol ou avec au moins un produit d'addition de l'oxyde d'éthylène et du polyglycérol,
- les esters résultant de la réaction du glycérol ou du polyglycérol avec au moins un produit d'addition de l'oxyde d'éthylène et d'un acide gras saturé ou insaturé,
- les esters partiels résultant de la réaction d'au moins un acide gras linéaire ou ramifié, saturé ou insaturé, d'acide ricinoléique ou d'acide 12-hydroxystéarique, avec au moins un polyol tel que le glycérol, le polyglycérol, le pentaérythritol, les alcools saccharidiques tels que le sorbitol, et en particulier les esters de polyglycérol,
- les esters résultant de la réaction du sorbitan avec au moins un acide gras linéaire ou ramifié, saturé ou insaturé,
- les esters de sorbitan modifiés éther, et en particulier les esters résultant iii) de la réaction du sorbitan avec au moins un produit d'addition de l'oxyde d'éthylène et d'un acide gras saturé ou insaturé ou iv) de la réaction d'au moins un acide gras saturé ou insaturé avec au moins un produit d'addition de l'oxyde d'éthylène et du sorbitan,
- les produits d'addition de l'oxyde d'éthylène avec l'huile de ricin et / ou l'huile de ricin hydrogénée,
- les phosphate trialkylés et les mono, di et triphosphate alkylés, et leurs mélanges,

ces composés satisfaisant aux paramètres de solubilité définis ci-dessus.

Le mot ester selon l'invention signifie un monoester, un diester, un triester et plus généralement un polyester.

De préférence, le dispersant hydrocarboné est choisi parmi les monoesters, les diesters et les esters résultant d'une estérification partielle, c'est à dire que l'ester final comporte une ou plusieurs fonctions -OH libre.

Avantageusement, le dispersant hydrocarboné est choisi parmi :

- les produits d'addition de 2 à 30 moles d'oxyde d'éthylène et / ou de 0 à 5 moles d'oxyde de propylène avec i) un alcool gras linéaire ou ramifié en $C_8$ à $C_{40}$, et mieux en $C_8$ à $C_{22}$ ou avec ii) un alkylphénol,
- les esters résultant de la réaction d'au moins un acide gras en $C_8$ à $C_{40}$, et mieux en $C_8$ à $C_{22}$, avec au moins un produit d'addition de 1 à 30 moles d'oxyde d'éthylène et du glycérol ou avec au moins un produit d'addition de 1 à 30 moles d'oxyde d'éthylène et du polyglycérol,
- les esters résultant de la réaction du glycérol ou du polyglycérol avec au moins un produit d'addition de 2 à 30 moles d'oxyde d'éthylène et d'un acide gras saturé ou insaturé en $C_8$ à $C_{40}$, et mieux en $C_8$ à $C_{22}$,
- les esters partiels résultant de la réaction d'au moins un acide gras linéaire ou ramifié, saturé ou insaturé en $C_8$ à $C_{40}$ et mieux en $C_8$ à $C_{22}$, d'acide ricinoléique ou d'acide 12-hydroxystéarique, avec le glycérol, le polyglycérol, le pentaérythritol ou le sorbitol,
- les esters résultant de la réaction du sorbitan avec au moins un acide gras linéaire ou ramifié, saturé ou insaturé en $C_8$ à $C_{40}$ et mieux en $C_8$ à $C_{22}$,
- les esters résultant iii) de la réaction du sorbitan avec au moins un produit d'addition de 2 à 30 moles d'oxyde d'éthylène et d'un acide gras saturé ou insaturé en $C_8$ à $C_{40}$ et mieux en $C_8$ à $C_{22}$ ou iv) de la réaction d'au moins un acide gras saturé ou insaturé en $C_8$ à $C_{40}$ et mieux en $C_8$ à $C_{22}$, avec au moins un produit d'addition de 2 à 30 moles d'oxyde d'éthylène et du sorbitan,
- les produits d'addition de 2 à 60 moles d'oxyde d'éthylène avec l'huile de ricin et / ou l'huile de ricin hydrogénée,
- les phosphate trialkylés et les mono, di et triphosphate alkylés,
- et leurs mélanges.

De manière préférentielle, le dispersant hydrocarboné est choisi parmi : l'alcool de myristyle oxyéthyléné à 15 groupements oxyde d'éthylène (ou OE) ($\delta_d$ = 17,33 $(J/cm^3)^{1/2}$ et $\delta_a$ = 9,28 $(J/cm^3)^{1/2}$), le monoisostéarate de polyglycérol-2 oxyéthyléné à 5 OE ($\delta_d$ = 17,34 $(J/cm^3)^{1/2}$ et $\delta_a$ = 12,22 $(J/cm^3)^{1/2}$), le diisostéarate de polyglyoérol-3 ($\delta_d$ = 16,96 $(J/cm^3)^{1/2}$ et $\delta_a$ = 10,4 $(J/cm^3)^{1/2}$), le monoisostéarate de glycérol ($\delta_d$ = 16,32 $(J/cm^3)^{1/2}$ et $\delta_a$ = 11,01 $(J/cm^3)^{1/2}$), le monoisostéarate de polyglyoérol-2 ($\delta_d$ = 17,03 $(J/cm^3)^{1/2}$ et $\delta_a$ = 13,25 $(J/cm^3)^{1/2}$), l'isostéarate de polyglycérol-3 ($\delta_d$ = 17,38 $(J/cm^3)^{1/2}$ et $\delta_a$ = 14,48 $(J/cm^3)^{1/2}$), l'isostéarate de polyglycérol-4 ($\delta_d$ = 17,57 $(J/cm^3)^{1/2}$ et $\delta_a$ = 15,37 $(J/cm^3)^{1/2}$), le monoisostéarate de polyglycérol-6 ($\delta_d$ = 17,86 $(J/cm^3)^{1/2}$ et $\delta_a$ = 16,61 $(J/cm^3)^{1/2}$), le monoisostéarate de polyglycérol-10 ($\delta_d$ = 18,22 $(J/cm^3)^{1/2}$ et $\delta_a$ = 18,41 $(J/cm^3)^{1/2}$), le monooléate de polyglycérol-2 ($\delta_d$ = 17,14 $(J/cm^3)^{1/2}$ et $\delta_a$ = 13,39 $(J/cm^3)^{1/2}$), l'isoetéarate de sorbitan ($\delta_d$= 17,33 $(J/cm^3)^{1/2}$ et $\delta_a$ = 13,56 $(J/cm^3)^{1/2}$), le monooléate de sorbitan ($\delta_d$ = 17,32 $(J/cm^3)^{1/2}$ et $\delta_a$ = 13,66 $(J/cm^3)^{1/2}$), le monooléate de sorbitan oxyéthyléné à 5 OE ($\delta_d$ = 17,56 $(J/cm^3)^{1/2}$ et $\delta_a$ = 12,47 $(J/cm^3)^{1/2}$), et leurs mélanges.

Avantageusement, le dispersant hydrocarboné est choisi parmi les esters partiels de polyglycérol et d'acide isostéarique, les esters partiels de polyglycérol et d'acide oléique, les esters partiels de sorbitan et d'acide oléique, et leurs mélanges.

Comme dispersant hydrocarboné utilisable préférentiellement dans la composition selon l'invention, on peut choisir le monoisostéarate de polyglycérol-2 tel que le Salacos 41 fabriqué ou commercialisé par la société Nisshin Oil

Mills, le diisostéarate de polyglycérol-3 tel que le Lameform TGI fabriqué ou commercialisé par la société Cognis, le monooléate de polyglycérol-2 tel que le Rylo PG 29 fabriqué ou commercialisé par la société Danisco Ingrédients, le monooléate de sorbitan tel que le Span 80 fabriqué ou commercialisé par la société Uniqema, et leurs mélanges.

Les quantités des différents ingrédients de la composition selon l'invention seront données en pourcentages en poids par rapport au poids total de la composition.

L'agent dispersant hydrocarboné représente de 0,5 à 40 %, de préférence de 3 à 20% et mieux de 5 à 15% du poids total de la composition.

Le composé siliconé non volatil utilisé dans la composition selon l'invention est un composé non aqueux et en particulier une huile cosmétique. Par "huile", on entend tout milieu non aqueux liquide et insoluble dans l'eau à température ambiante (25°C) et pression atmosphérique (760mm de Hg ou $1{,}01.10^5$Pa).

De manière préférentielle, le composé siliconé non volatil présente une viscosité allant de 5 à 1 000 000 cSt à 25°C, de préférence allant de 10 à 500 000 cSt et mieux de 10 à 5 000 cSt.

A titre d'exemples de composés siliconés non volatils utilisables dans la composition de l'invention, on peut citer les polydiméthylsiloxanes (PDMS), les silicones phénylées, les polyalkylméthylsiloxanes, les résines de silicones telles celles décrites dans les documents JP-A-62-61911, JP-A-61-65809 et EP-A-602905, les silicones fluorées et leurs mélanges.

En particulier, ces composés siliconés non volatils sont choisis parmi les polydiméthylsiloxanes non volatils; les polydiméthylsiloxanes comportant des groupements alkyle, alcoxy ou phényle, pendant ou en bout de chaîne siliconée, groupements ayant de 2 à 24 atomes de carbone ; les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ; les silicones fluorées comportant un groupement fluoré pendant ou en bout de chaîne siliconée ayant de 1 à 12 atomes de carbone dont tout ou partie des hydrogènes est substitué par des atomes de fluor ; les résines de silicone ; les gommes de silicone dont les diméthiconols; et leurs mélanges.

Le composé siliconé non volatil peut représenter de 0,5 à 90 %, de préférence de 5 à 60 % et mieux de 10 à 50 % du poids total de la composition.

La composition selon l'invention comprend également un polymère liposoluble non siliconé.

Ce polymère liposoluble non siliconé a notamment une masse moléculaire allant de 400 à 500000 g/mol et de préférence de 500 à 100000 g/mol.

Le polymère liposoluble non siliconé est choisi parmi les polymères vinyliques, les polyéthers liposolubles, les polyesters non réticulés, et leurs mélanges.

Ce polymère liposoluble non siliconé peut être choisi parmi :

a) les polymères vinyliques et notamment :

- les homopolymères d'oléfines non cristallins ramifiés,
- les copolymères d'oléfines non ramifiés, non cristallins,
- les homopolymères et copolymères de diènes hydrogénés non cristallins,
- les oligomères linéaires ou ramifiés, homo ou copolymères de (méth)acrylates d'alkyles ayant de préférence un groupement alkyle en $C_8$-$C_{30}$ et une masse molaire inférieure ou égale à 10000g/mol, les oligomères homo et copolymères de (méth)acrylates d'alkyles perfluorés, avec de préférence un groupement alkyle en $C_8$-$C_{30}$. et une masse molaire inférieure ou égale à 10000 g/mol, et mieux inférieure ou égale à 8000 g/mol,
- les oligomères homo et copolymères d'esters vinyliques ayant des groupements alkyles en $C_8$-$C_{30}$ et une masse molaire inférieure ou égale à 10000 g/mol, et mieux inférieure ou égale à 8000 g/mol,
- les oligomères homo et copolymères de vinyléthers ayant des groupements alkyles en $C_8$-$C_{30}$, ayant une masse molaire inférieure ou égale à 10000 g/mol, et mieux inférieure ou égale à 8000 g/mol,

b) les polyéthers liposolubles résultant de la polyétherification entre un où plusieurs diols en $C_2$-$C_{100}$, de préférence en $C_2$-$C_{50}$,

c) les polyesters non réticulés résultant de la polycondensation entre un diacide ou un polyacide carboxylique linéaire ou ramifié en $C_4$-$C_{50}$ et un diol ou un polyol en $C_2$-$C_{50}$,

et leurs mélanges.

A titre de polymère liposoluble non siliconé utilisable dans la présente invention, on peut citer :

parmi les homopolymères d'oléfines :

- les polybutylènes tels que l'Indopol H-100 (de masse molaire ou MM=965 g/mol), l'Indopol H-300 (MM=1340 g/mol) ou l'Indopol H-1500 (MM=2160 g/mol) fabriqués ou commercialisés par la société Amoco,

- les polyisobutylènes hydrogénés tels que Panalane H-300E (MM=1340 g/mol) fabriqué ou commercialisé par la société Amoco, le Viseal 20000 (MM=6000 g/mol) fabriqué ou commercialisé par la société Synteal, le Rewopal PIB 1000 (MM=1000g/mol) fabriqué ou commercialisé par la société Witco,
- les polydéoènes et les polydéoènes hydrogénés tels que le Puresyn 8 (MM=635 g/mol), le Puresyn 10 (MM=723 g/mol) ou le Puresyn 150 (MM=9200 g/mol) fabriqués ou commercialisés par la société Mobil Chemicals ;

parmi les copolymères d'oléfines non ramifiées :

- les copolymères vinyl pyrrolidone (VP)/oléfines ayant un nombre de carbones allant de 8 à 30, de préférence de 10 à 30 comme le copolymère vinyl pyrrolidone /1-hexadécène fabriqué ou commercialisé par la société ISP sous le nom Antaron V-216 (MM=7300 g/mol) ;

parmi les homopolymères et copolymères de diènes, hydrogénés et non cristallins: le polybutadiène hydrogéné, le polyisoprène hydrogéné,
Parmi les oligomères homo et copolymères d'esters vinyliques :

- le polylaurate de vinyle (homopolymère de dodécanoate d'éthényle) tel que le Mexomère PP fabriqué ou commercialisé par la société Chimex,
- le copolymère acétate de vinyle/stéarate d'allyl tel que le Mexomère PQ fabriqué ou commercialisé par la société Chimex ;

parmi les polyéthers liposolubles :

- le copolymère PEG-45/dodécyle glycol (polymère bloc de polyoxyethylène/polydodécyle glycol) tel que l'ELFACOS ST 9 fabriqué ou commercialisé par la société Akzo Nobel ; et leurs mélanges.

Le polymère liposoluble non siliconé peut représenter de 1 à 98,5 %, de préférence de 2 à 85 %, mieux de 5 à 70%, et encore mieux de 5 à 60% du poids total de la composition.

Avantageusement, la composition de l'invention peut comprendre, en outre, au moins une matière colorante qui peut être choisie parmi les colorants solubles ou dispersibles dans la composition, les pigments, les nacres ou pigments nacrés et leurs mélanges. Les colorants sont de pnéférence des colorants liposolubles, bien que les colorants hydrosolubles puissent être utilisés. Cette matière colorante peut représenter de 0,001 à 98 %, de préférence de 0,5 à 85% et mieux de 1 à 60 % du poids total de la composition.

Pour une composition sous forme de pâte ou coulée telle que les rouges à lèvres, les fonds de teint ou les produits de maquillage du corps, la matière colorante représente en générale de 0,001 à 60%, de préférence de 2 à 40 % et mieux de 5 à 30 % du poids total de la composition.

Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).

De préférence, la composition de l'invention, comprend une phase particulaire pouvant représenter de 0,001 à 50 % du poids total de la composition, de préférence de 0,01 à 40 %, mieux de 0,5 à 25%, et encore mieux de 0,05 à 30 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 30 % (si présents) du poids de la composition finale, et de préférence à raison de 2 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % (si présentes) du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica

titane coloré, les pigments goniochromatiques et par exemple les pigments multicouches interférentiels.

Les charges peuvent être présentes à raison de 0,001 à 35 % (si présentes) du poids total de la composition, de préférence 0,5 à 15 %. On peut notamment citer le talc, le mica, le kaolin, la lauroyl lysine, les poudres de polyamide telles que le Nylon® (Orgasol notamment) et de polyéthylène, les poudres de polytétrafluoroéthylène (Téflon®), l'amidon, le nitrure de bore, des microsphères de copolymères telles que l'Expancel® (Nobel Industrie), le Polytrap® (Dow Corning), le Polypore® L 200 (Chemdal Corporation) et les microbilles de résine de silicone (Tospearl ® de Toshiba, par exemple).

La composition selon l'invention peut contenir au moins un composé non aqueux additionnel différent du composé siliconé non volatil, du polymère liposoluble non siliconé et de l'agent dispersant hydrocarboné, choisi parmi les huiles, les corps gras pâteux à température ambiante, les cires, les gommes, les résines, et leurs mélanges.

En particulier, elle contient, en outre, au moins une cire. Par "cire" au sens de la présente invention, on entend un composé gras lipophile, solide à température ambiante (25°C), à changement d'état solide/liquide réversible, ayant une température de fusion supérieure à 30°C, et mieux supérieure à 45°C, pouvant aller jusqu'à 200° C, une dureté supérieure à 0,5 MPa, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Comme cire utilisable dans l'invention, on peut citer celles généralement utilisées dans le domaine cosmétique : elles sont notamment d'origine naturelle comme la cire d'abeilles, la cire de Camauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ou l'huile de ricin hydrogénée ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 30°C et mieux à 45°C, les cires de silicones comme lesalkyl- ou alkoxydiméthicones ayant une chaîne alkyle ou alcoxy de 10 à 45 atomes de carbone, les esters de poly(di)méthylsiloxane solide à 30°C dont la chaîne ester comporte au moins 10 atomes de carbone ou encore le tétrastéarate de di-(triméthylol-1,1,1 propane) fabriqué ou commercialisé par la société Hétérène sous la dénomination HEST 2T- 4S ; et leurs mélanges.

La nature et la quantité des gommes, corps pâteux ou cires sont fonction des propriétés mécaniques et des textures recherchées. A titre indicatif, la composition peut contenir de 0,01 à 50 % en poids de cires, par rapport au poids total de la composition, de préférence de 2 à 40 %, et mieux de 5 à 30 %.

Les huiles additionnelles peuvent être des huiles hydrocarbonées et/ou fluorées. Ces huiles peuvent être d'origine animale, végétale, minérale ou synthétique. "Par huile hydrocarbonée", on entend une huile comportant principalement des atomes de carbone et d'hydrogène et éventuellement une ou plusieurs fonctions choisies parmi les fonctions hydroxyle, ester, éther, carboxylique.

A titre d'exemple d'huile utilisable dans l'invention, on peut citer :

- les huiles hydrocarbonées d'origine animale telles que le perhydrosqualène ;
- les huiles hydrocarbonées d'origine végétale telles que les triglycérides liquides d'acides gras ayant de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stéarineries Dubois ou ceux vendus sous les dénominations Miglyol 810, 812 et 818 par la société Dynamit Nobel, l'huile de jojoba, de beurre de karité, le squalane d'origine synthétique ou végétale;
- les hydrocarbures linéaires ou ramifiés, d'origine minérale ou synthétique tels que les huiles de paraffine et leurs dérivés, la vaseline ;
- les esters et les éthers de synthèse notamment d'acides gras comme les huiles de formule $R_1COOR_2$ dans laquelle $R_1$ représente le reste d'un acide gras supérieur comportant de 1 à 40 atomes de carbone et $R_2$ représente une chaîne hydrocarbonée contenant de 1 à 40 atomes de carbone avec $R_1 + R_2 \geq 10$ comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de penlaérythrityle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées comme le méthoxynonafluorobutane ;
- leurs mélanges.

Les huiles additionnelles de la composition peuvent représenter de 0,1 % à 90 % du poids total de la composition, de préférence de 5 à 60 % et mieux de 10 à 50 %.

La composition de l'invention peut comprendre, en outre, au moins un additif usuellement utilisé dans le domaine concerné, tel que des antioxydants, des conservateurs, des neutralisants, des gélifiants lipophiles ou de composés non aqueux liquides, des dispersants, des actifs cosmétiques, et leurs mélanges. Ces additifs, peuvent être présents dans la composition à raison de 0,0005 à 20% du poids total de la composition et mieux de 0,001 à 10%.

Par « actif cosmétique », on entend un composé lipophile ou hydrophile apportant un bénéfice aux matières kératiniques et plus spécialement à la peau et aux lèvres.

Comme actif cosmétique utilisable dans l'invention, on peut citer les vitamines A, E, C, $B_3$, F, les provitamines comme le D-panthénol, les actifs apaisants comme l'$\alpha$-bisabolol, l'aloe vers, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, les filtres solaires, et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, ou sous la forme compacté par exemple sous forme de coupelle utilisable par contact direct ou à l'éponge ou encore dans une bouillotte. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple ou encore de gel, de crème plus ou moins fluide. Elles peuvent alors constituer des fonds de teint ou des rouges à lèvres, des brillants à lèvres (gloss en terminologie anglo-saxonne), des produits solaires ou de coloration de la peau.

Les compositions de l'invention peuvent alors se présenter notamment sous forme de gel huileux, de liquide huileux, de pâte ou de stick ou encore sous forme de dispersion vésiculaire contenant des lipides ioniques et/ou non ioniques. Ces formes galéniques sont préparées selon les méthodes usuelles des domaines considérés.

La composition selon l'invention peut se présenter sous la forme d'une composition, colorée ou non de soin de la peau, sous forme d'une composition de protection solaire ou de démaquillage ou encore sous forme d'une composition hygiénique. Si elle contient des actifs cosmétiques, elle peut alors être utilisée comme base de soin ou de traitement non thérapeutique pour la peau comme les mains ou le visage ou pour les lèvres (baumes à lèvres, protégeant les lèvres du froid et/ou du soleil et/ou du vent).

La composition de l'invention peut également se présenter sous la forme d'un produit de maquillage coloré de la peau, en particulier du visage comme un fond de teint, un blush, un fard à joues ou à paupières, de maquillage du corps comme un produit de tatouage semi-permanent, ou de maquillage des lèvres comme un rouge ou un brillant à lèvres, présentant éventuellement des propriétés de soin ou de traitement non thérapeutique, un produit de maquillage des phanères comme par exemple un vernis à ongles, un mascara, un eyeliner.

De préférence, la composition selon l'invention se présente sous forme de rouge à lèvres ou de brillant à lèvres.

Bien entendu la composition de l'invention doit être physiologiquement acceptable (cosmétiquement acceptable), à savoir non toxique et susceptible d'être appliquée sur la peau, les phanères ou les lèvres d'êtres humains.
Par « cosmétiquement acceptable », on entend agréable de goût, de toucher, d'aspect et/ou d'odeur.

L'invention est illustrée plus en détail dans les exemples suivants. Les pourcentages sont des pourcentages massiques.

## Exemples 1 et 2 : Sticks de rouges à lèvres

Les compositions figurant dans le tableau (1) ci-après ont été réalisées. La composition de l'exemple 1 (invention) comprend, comme agent dispersant selon l'invention, le monooléate de sorbitan ($\delta d$ = 17,32 $(J/cm^3)^{1/2}$ et $\delta a$ = 13,66 $(J/cm^3)^{1/2}$), fabriqué ou commercialisé par la société Uniqema sous la référence Span 80 V.
Dans la composition de l'exemple 2 (comparatif), les 13,4% de monooléate de sorbitan ont été substitués par 10% de triisostéarate de polyglycérol-2 fabriqué ou commercialisé par la société Nisshin Oil Mills sous la référence Salacos 43 ($\delta d$ = 16,7 $(J/cm^3)^{1/2}$ et $\delta a$ = 6,69 $(J/cm^3)^{1/2}$).

**Tableau (I)**

| Phase | | Exemple 1 (invention) | Exemple 2 (comparatif) |
|---|---|---|---|
| A | Triméllitate de tridécyle | 4,4 | 4,4 |

(suite)

| Phase | | Exemple 1 (invention) | Exemple 2 (comparatif) |
|---|---|---|---|
| | Copolymère vinylpyrrolidone/1-hexadécène commercialisé ou fabriqué par la société ISP sous la référence Antaron V-216 | 1,6 | 1,6 |
| | Copolymère vinylpyrrolidone/1-eicosène commercialisé ou fabriqué par la société ISP sous la référence Antaron V-220 | 0,8 | 0,8 |
| | Bis diglycéryl polyacyladipate-2 | 1,6 | 1,6 |
| | BHT | 0,04 | 0,04 |
| | Triisostéarate de polyglycérol-2 | 0 | 13,4 |
| | Monooléate de sorbitan | 13,4 | - |
| | Isononanoate d'isononyle | 10 | 10 |
| **B-** | Cire de polyéthylène (MM = 500 g/mol). | 6,6 | 6,6 |
| | Stéarate d'octacosanyle | 5,5 | 5,5 |
| **C-** | Red 21 | 0,06 | 0,06 |
| | Red 7 | 0,2 | 0,2 |
| | Iron oxydes (CI 77491 et CI 77499) | 2,2 | 2,2 |
| **C'-** | Copolymère di méthacrylate d'éthylène glycol/méthacrylate de lauryle fabriqué ou commercialisé sous la référence Polytrap 603 par la société Advanced Polymer Systems | 1 | 1 |
| | N-lauroyl L-lysine | 2,5 | 2,5 |
| | Kaolin | 5 | 5 |
| **D-** | Isoparaffine hydrogénée fabriqué ou commercialisé sous la référence Parléam par la société Nippon Oil and Fats | qsp 100 | qsp 100 |
| | Polyisobutène hydrogéné fabriqué ou commercialisé sous la référence Panalane H-300E par la société Amoco Chemical | 8,5 | 8,5 |
| | Phényltrimethicone commercialisé ou fabriqué par la société Dow Corning sous la référence DC 556 | 12,7 | 12,7 |
| | Silice pyrogénée | 3 | 3 |
| **E-** | Mica oxyde de titane | 1,8 | 1,8 |

<u>Mode opératoire :</u>

Les pigments (phase C) et les charges (phase C') sont broyés dans la phase A.
Parallèlement, un gel de silice est préparé (phase D) en mélangeant la silice dans l'isoparaffine hydrogénée, la phényltrimethicone et le polyisobutène hydrogéné.
Puis le broyat (phases A + C + C') ainsi que le gel de silice (phase D) et les cires (phase B), sont ajoutés dans un poêlon et chauffés à 100°C pendant 2 heures et homogénéisée.
Enfin, la nacre (phase E) est ajoutée au mélange que l'on coule dans un moule approprié à 42°C. Le moule est ensuite placé à - 20°C pendant une demi-heure, puis on procède au démoulage des sticks.

<u>Evaluation cosmétique</u> :

La tenue des deux formules a été évaluée à l'aide de méthodes instrumentales et sensorielles sur un panel

de 12 personnes qualifiées qui ont appliqué chacune des formules l'une après l'autre.
L'évaluation de la tenue se déroule de la façon suivante :

- dans un premier temps, une évaluation de la tenue est réalisée une heure après l'application de la formule sur les lèvres.
- dans un second temps, la tenue est évaluée après une série d'épreuves consistant à faire deux « bises » sur un mouchoir en papier, boire une boisson chaude puis une boisson froide et manger 4 bouchées d'un sandwich et une pomme.
  La tenue instrumentale est évaluée sur une échelle allant de 1 à 100 : 1 correspond à une formule qui ne tient pas du tout et 100 à une formule qui tient très bien. La différence entre deux résultats est significative si elle est supérieure ou égale à 10.

La migration, la brillance et le confort ont aussi été évalués par les 12 personnes:

- la brillance a été évaluée juste après l'application de la formule puis au bout d'une heure
- la migration et le confort ont été évalués au bout d'une heure.

La composition de l'exemple 1 selon l'invention possède de meilleures propriétés de tenue que la composition de l'exemple 2 (la tenue a été évaluée à 71 pour la composition de l'exemple 1 selon l'invention contre une valeur de 59 pour la composition de l'exemple 2), tout en étant de brillance, de confort et de migration équivalentes. De plus, la brillance du film de la composition de l'exemple 1 persisté plus longtemps.

**Exemples 3 et 4 : Sticks de rouges à lèvres**

Les inventeurs ont comparé les propriétés de deux compositions selon l'invention et l'art antérieur. La composition de l'exemple 3 selon l'invention contient un agent dispersant hydrocarboné qui est le monoisostéarate de polyglycérol-2 (fabriqué ou commercialisé par la société Nisshin Oils Mills sous la référence Salacos 41) et la composition de l'exemple 4 (comparatif) contient un agent dispersant siliconé qui est le cétyl diméthicone copolyol (fabriqué ou commercialisé par la société Goldschmidt sous la référence Abil EM 90 Desodorise).

**Tableau (II)**

| Phase | | Exemple 3 (invention) | Exemple 4 (comparatif) |
|---|---|---|---|
| A - | Triméllitate de tridécyle | 4,4 | 4,4 |
| | Copolymère vinylpyrrolidone/1-hexadecene commercialisé ou fabriqué par la société ISP sous la référence Antaron V-216 | 1,6 | 1,6 |
| | Copolymère vinylpyrrolidone/1-eicosène commercialisé ou fabriqué par la société ISP sous la référence Antaron V-220 | 0,8 | 0,8 |
| | Bis diglycéryl polyacyladipate-2. | 1,6 | 1,6 |
| | BHT | 0,04 | 0,04 |
| | Triisostéarate de polyglycéryle-2 | 3,4 | 3,4 |
| | Cétyl diméthicone copolyol | - | 10 |
| | Monoisostéarate de polyglycérol-2 | 10 | - |
| | Isononanoate d'isononyle | 10 | 10 |
| B- | Cire de polyéthylène (MM= 500 g/mol) | 6,6 | 6,6 |
| | Stéarate d'octa-cosanyle | 5,5 | 5,5 |
| C- | Red 21 | 0,06 | 0,06 |
| | Red 7 | 0,2 | 0,2 |
| | Iron oxydes | 2,2 | 2,2 |

(suite)

| Phase | | Exemple 3 (invention) | Exemple 4 (comparatif) |
|---|---|---|---|
| C'- | Copolymère di méthacrylate d'éthylène glycol/méthacrylate de lauryle fabriqué ou commercialisé sous la référence Polytrap 603 par la société Advanced Polymer Systems | 1 | 1 |
| | N-lauroyl L-lysine | 2,5 | 2,5 |
| | Kaolin | 5 | 5 |
| D- | Isoparaffine hydrogénée fabriqué ou commercialisé sous la référence Parléam par la société Nippon Oil and Fats | qsp 100 | qsp 100 |
| | Polyisobutène hydrogéné fabriqué ou commercialisé sous la référence Panalane H-300E par la société Amoco Chemical | 8,5 | 8,5 |
| | Phényltrimethicone commercialisé ou fabriqué par la société Dow Corning sous la référence DC 556 | 12,7 | 12,7 |
| | Silice pyrogénée | 3 | 3 |
| E- | Mica oxyde de titane | 1,8 | 1,8 |

Le mode opératoire est le même que celui des exemples 1 et 2 ci-dessus.

Evaluation cosmétique

- La tenue, la migration, la brillance et le confort ont été évalués selon les mêmes méthodes que celles des exemples 1 et 2.

La composition de l'exemple 3 selon l'invention a été jugée brillante, confortable, peu migrante et possède une tenue nettement supérieure à celle de la composition de l'exemple 4 (la tenue après épreuve a été évaluée à 71 pour la composition 3 selon l'invention contre 49 pour la composition de l'exemple 4).

## Revendications

**1.** Composition anhydre de soin ou de maquillage des matières kératiniques, comprenant un milieu cosmétiquement acceptable contenant

- au moins un composé siliconé non volatile ledit composé siliconé non rotatif étant une huile cosmétique
- au moins un polymère liposoluble non siliconé et
- au moins un agent dispersant hydrocarboné qui présente les paramètres de solubilité δd et δa satisfaisant aux conditions suivantes: $16{,}2 \leq \delta_d \leq 20$ $(J/cm^3)^{1/2}$ et $9{,}1 \leq \delta_a \leq 20$ $(J/cm^3)^{1/2}$, ledit agent dispersant comportant des atomes de carbone et d'hydrogène et une ou plusieurs fonctions choisies parmi les fonctions hydroxyle, ester, éther, carboxylique ou amide, ledit agent dispersant hydrocarbone représentant de 0,5 à 40% du poids total de la composition

et ladite composition comprenant moins de 10% et huile volatile par rapport au poids total de la composition.

**2.** Composition selon la revendication 1, **caractérisée en ce qu**'elle contient moins de 5% d'huile volatile par rapport au poids total de la composition, et mieux moins de 2%.

**3.** Composition selon l'une des revendications précédentes, **caractérisée en ce que** l'agent dispersant hydrocarboné présente les paramètres de solubilité δd et δa satisfaisant aux conditions suivantes : $16{,}3 \leq \delta_d \leq 19 (J/cm^3)^{1/2}$ et $10 \leq \delta_a \leq 18{,}1 (J/cm^3)^{1/2}$

**4.** Composition selon l'une des revendications précédentes, **caractérisée en ce que** l'agent dispersant hydrocarboné présente les paramètres de solubilité δd et δa satisfaisant aux conditions suivantes : $16,9 \leq \delta_d \leq 18 (J/cm^3)^{1/2}$ et $13 \leq \delta_a \leq 14,5$ $(J/cm^3)^{1/2}$.

**5.** Composition selon l'une des revendications précédentes, **caractérisée en ce que** l'agent dispersant hydrocarboné est fluide à température ambiante (25°C) et/ou présente un indice de réfraction ≥ 1,45 à 20°C.

**6.** Composition selon l'une des revendications précédentes, **caractérisée en ce que** l'agent dispersant hydrocarboné présente une structure chimique comportant au moins un groupement polaire choisi parmi -COOH ; -OH ; oxyde d'éthylène : $-(O-CH_2-CH_2-)$; oxyde de propylène

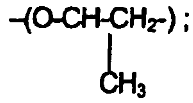

$-PO_4$ ; NHR ; $NR_1R_2$ avec $R_1$, $R_2$ formant éventuellement un cycle et représentant un radical alkyle ou alkoxy linéaire ou ramifié en $C_1$ à $C_{20}$ ou

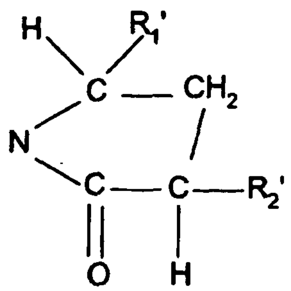

avec $R_1'$ et $R_2'$ qui peuvent être égal à H ou à une chaîne alkyle ou alkoxy linéaire ou ramifiée en $C_1$ à $C_{20}$.

**7.** Composition selon l'une des revendications précédentes, **caractérisée en ce que** le dispersant hydrocarboné est choisi parmi:

- les alcools gras modifiés éther et en particulier les produits d'addition de l'oxyde d'éthylène et /ou de l'oxyde de propylène avec i) un alcool gras linéaire ou ramifié ou avec ii) un alkylphénol,- les esters résultant de la réaction d'au moins un acide gras avec au moins un produit d'addition de l'oxyde d'éthylène et du glycérol ou avec au moins un produit d'addition de l'oxyde d'éthylène et du polyglycérol,
- les esters résultant de la réaction du glycérol ou du polyglycérol avec au moins un produit d'addition de l'oxyde d'éthylène et d'un acide gras saturé ou insaturé,
- les esters partiels résultant de la réaction d'au moins un acide gras linéaire ou ramifié, saturé ou insaturé, d'acide ricinoléique ou d'acide 12-hydroxystéarique, avec au moins un polyol tel que le glycérol, le polyglycérol, le pentaérythritol, les alcools saccharidiques tels que le sorbitol, et en particulier les esters de polyglycérol,
- les esters résultant de la réaction du sorbitan avec au moins un acide gras linéaire ou ramifié, saturé ou insaturé,
- les esters de sorbitan modifiés éther, et en particulier les esters résultant iii) de la réaction du sorbitan avec au moins un produit d'addition de l'oxyde d'éthylène et d'un acide gras saturé ou insaturé ou iv) de la réaction d'au moins un acide gras saturé ou insaturé avec au moins un produit d'addition de l'oxyde d'éthylène et du sorbitan,
- les produits d'addition de l'oxyde d'éthylène avec l'huile de ricin et /ou l'huile de ricin hydrogénée,
- les phosphate trialkylés et les mono, di et triphosphate alkylés,

et leurs mélanges.

**8.** Composition selon l'une des revendications précédentes, **caractérisée en de que** le dispersant hydrocarboné est choisi parmi les monoesters, les diesters et les esters résultant d'une estérification partielle.

**9.** Composition selon l'une des revendications précédentes, **caractérisée en ce que** le dispersant hydrocarboné est

choisi parmi:

- les produits d'addition de 2 à 30 moles d'oxyde d'éthylène et / ou de 0 à 5 moles d'oxyde de propylène avec i) un alcool gras linéaire ou ramifié en $C_8$ à $C_{40}$, et mieux en $C_8$ à $C_{22}$ ou avec ii) un alkylphénol,
- les esters résultant de la réaction d'au moins un acide gras en $C_8$ à $C_{40}$, et mieux en $C_8$ à $C_{22}$, avec au moins un produit d'addition de 1 à 30 moles d'oxyde d'éthylène et du glycérol ou avec au moins un produit d'addition de 1 à 30 moles d'oxyde d'éthylène et du polyglycérol,
- les esters résultant de la réaction du glycérol ou du polyglycérol avec au moins un produit d'addition de 2 à 30 moles d'oxyde d'éthylène et d'un acide gras saturé ou insaturé en $C_8$ à $C_{40}$, et mieux en $C_8$ à $C_{22}$,
- les esters partiels résultant de la réaction d'au moins un acide gras linéaire ou ramifié, saturé ou insaturé en $C_8$ à $C_{40}$ et mieux en $C_8$ à $C_{22}$, d'acide ricinoléique ou d'acide 12-hydroxystéarique, avec leglycérol, le polyglycérol, le pentaérythritol ou le sorbitol,
- les esters résultant de la réaction du sorbitan avec au moins un acide gras linéaire ou ramifié, saturé ou insaturé en $C_8$ à $C_{40}$ et mieux en $C_8$ à $C_{22}$,
- les esters résultant iii) de la réaction du sorbitan avec au moins un produit d'addition de 2 à 30 moles d'oxyde d'éthylène et d'un acide gras saturé ou insaturé en $C_8$ à $C_{40}$ et mieux en $C_8$ à $C_{22}$ ou iv) de la réaction d'au moins un acide gras saturé ou insaturé en $C_8$ à $C_{40}$ et mieux en $C_8$ à $C_{22}$, avec au moins un produit d'addition de 2 à 30 moles d'oxyde d'éthylène et du sorbitan,
- les produits d'addition de 2 à 60 moles d'oxyde d'éthylène avec l'huile de ricin et /ou l'huile de ricin hydrogénée,
- les phosphate trialkylés et les mono, di et triphosphate alkylés,
- et leurs mélanges.

**10.** Composition selon l'une des revendications précédentes, **caractérisée en ce que** le dispersant hydrocarboné est choisi parmi : l'alcool de myristyle oxyéthyléné à 15 OE, le monoisostéarate de polyglycérol-2 oxyéthyléné à 5 OE, le diisostéarate de polyglycérol-3, le monoisostéarate de glycérol, le monoisostéarate de polyglycérol-2, l'isostéarate de polyglycérol-3, l'isostéarate de polyglycérol-4, le monoisostéarate de polyglycérol-6, le monoisostéarate de polyglycérol-10, le monooléate de polyglycérol-2, l'isostéarate de sorbitan, le monooléate de sorbitan, le monooléate de sorbitan oxyéthyléné à 5 OE, et leurs mélanges.

**11.** Composition selon l'une des revendications précédentes, **caractérisée en ce que** le dispersant hydrocarboné est choisi parmi les esters partiels de polyglycérol et d'acide isostéarique, les esters partiels de polyglycérol et d'acide oléique, les esters partiels de sorbitan et d'acide oléique, et leurs mélanges.

**12.** Composition selon l'une des revendications précédentes, **caractérisée en ce que** le dispersant hydrocarboné est choisi parmi le diisostéarate de polyglycérol-3, le monoisostéarate de polyglycérol-2, le monooléate de polyglycérol-2, le monooléate desorbitan, et leurs mélanges.

**13.** Composition selon l'une des revendications précédentes, **caractérisée en ce que** l'agent dispersant hydrocarboné représente de 3 à 20% et mieux de 5 à 15% du poids total de la composition.

**14.** Composition selon la revendication précédente, dans laquelle le composé siliconé non volatil présente une viscosité allant de 5 à 1000 000 cSt à 25°C, de préférence allant de 10 à 500 000 cSt et mieux de 10 à 5 000 cSt.

**15.** Composition selon l'une des revendications précédentes, dans laquelle le composé siliconé non volatil est choisi parmi les polydiméthylsiloxanes non volatils ; les polydiméthylsiloxanes comportant des groupements alkyle, alcoxy ou phényle, pendant ou en bout de chaîne siliconée, groupements ayant de 2 à 24 atomes de carbone ; les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates; les silicones fluorées comportant un groupement fluoré pendant ou en bout de chaîne siliconée ayant de 1 à 12 atomes de carbone dont tout ou partie des hydrogènes est substitué par des atomes de fluor gommes de silicone ; et leurs mélanges.

**16.** Composition selon l'une des revendications précédentes, **caractérisée en ce que** le composé siliconé non volatil représente de 0,5 à 90 %, de préférence de 5 à 60 % et mieux de 10 à 50 % du poids total de la composition.

**17.** Composition selon l'une des revendications précédentes, **caractérisée en ce que** le polymère liposoluble non siliconé a une masse moléculaire allant de 400 à 500000 g/mol et de préférence de 500 à 100000 g/mol.

**18.** Composition selon l'une des revendications précédentes, **caractérisée en ce que** le polymère liposoluble non

siliconé est choisi parmi les polymères vinyliques, les polyéthers liposolubles, les polyesters non réticulés, et leurs mélanges.

**19.** Composition selon l'une des revendications précédentes, **caractérisée en ce que** le polymère liposoluble non siliconé est choisi parmi :

- les homopolymères d'oléfines non cristallins ramifiés, les copolymères d'oléfines non ramifiés, les homopolymères et copolymères de diènes hydrogénés non cristallins, les oligomères linéaires ou ramifiés, homo ou copolymères de (méth)acrylates d'alkyles ayant de préférence un groupement alkyle en $C_8$-$C_{30}$ et une masse molaire inférieure ou égale à 10000g/mol, les oligomères homo et copolymères de (méth)acrylates d'alkyles perfluorés ayant une masse molaire inférieure ou égale à 10000 g/mol, et mieux inférieure ou égale à 8000 g/mol, les oligomères homo et copolymères d'esters vinyliques ayant des groupements alkyles en $C_8$-$C_{30}$ et une masse molaire inférieure ou égale à 10000 g/mol, et mieux inférieure ou égale à 8000 g/mol, les oligomères homo et copolymères de vinyléthers ayant des groupements alkyles en $C_8$-$C_{30}$, ayant une masse molaire inférieure ou égale à 10000 g/mol, et mieux inférieure ou égale à 8000 g/mol,- les polyéthers liposolubles résultant de la polyétherification entre un ou plusieurs diols en $C_2$-$C_{100}$, de préférence en $C_2$-$C_{50}$,
- les polyesters non réticulés résultant de la polycondensation entre un diacide ou un polyacide carboxylique linéaire ou ramifié en $C_4$-$C_{50}$ et un diol où un polyol en $C_2$-$C_{50}$,

et leurs mélanges.

**20.** Composition selon l'une des revendications précédentes, **caractérisée en ce que** le polymère liposoluble est choisi parmi les polybutylènes, les polyisobutylènes hydrogènes, les polydécènes, les polydécènes hydrogènes, les copolymères vinyl pyrrolidone (VP)/oléfines ayant un nombre de carbones allant de 8 à 30, de préférence de 10 à 30, le polybutadiène hydrogéné, le polyisoprène hydrogéné, le polylaurate de vinyle, le copolymère acétate de vinyle/stéarate d'allyl, le copolymère PEG-45/dodecyle glyool, et leurs mélanges.

**21.** Composition selon l'une des revendications précédentes, **caractérisée en ce que** le polymère liposoluble représente de 1 à 98,5 %, de préférence de 2 à 85 %, mieux de 5 à 70%, et encore mieux de 5 à 60% du poids total de la composition.

**22.** Composition selon l'une des revendications précédentes, **caractérisée en ce qu'**elle contient au moins une matière colorante.

**23.** Composition selon la revendication précédente, **caractérisée en ce que** la matière colorante est choisie parmi les colorants solubles ou dispersibles dans la composition, les pigments, les nacres et leurs mélanges.

**24.** Composition selon la revendication 22 ou 23, **caractérisée en ce que** la matière colorante représente de 0,5 à 50%, de préférence de 2 à 40 % et mieux de 5 à 30%, par rapport au poids total de la composition.

**25.** Composition selon l'une des revendications préoedentes, **caractérisée en ce qu**'elle contient une phase particulaire.

**26.** Composition selon l'une des revendications précédentes, **caractérisée en ce qu**'elle contient, en outre, au moins une cire.

**27.** Composition selon la revendication précédente, **caractérisée en ce que** la cire représente de 0,01 à 50%, de préférence de 2 à 40%, et mieux de 5 à 30% du poids total de la composition.

**28.** Composition selon l'une des revendications précédentes, **caractérisée en ce qu**'elle contient, en outre, au moins un additif choisi parmi les antioxydants, les conservateurs, les neutralisants, les gélifiants lipophiles ou de composés non aqueux liquides, les dispersants, les actifs cosmétiques, et leurs mélanges.

**29.** Composition selon l'une des revendications précédentes, **caractérisée en ce qu'**elle contient au moins un actif cosmétique choisi parmi les vitamines (A, E, C, $B_3$, F) les provitamines, les actifs apaisants, les extraits de plantes ou les huiles essentielles, les agents protecteurs ou restructurants, les actifs fraîcheur, les émollients, les hydratants, les actifs antirides, les acides gras essentiels, et leurs mélanges.

**30.** Composition selon l'une des revendications précédentes, **caractérisée en ce qu**'elle se présente sous forme d'un

produit coulé ou compacté.

**31.** Composition selon l'une des revendications précédentes, **caractérisée en ce qu'**elle se présente sous forme d'un rouge à lèvres ou d'un brillant à lèvres.

**32.** Procédé cosmétique pour conférer à un film de composition cosmétique anhydre des propriétés de tenue de la couleur, de brillance, de confort et/ou de non-migration, consistant à introduire dans ladite composition au moins composé siliconé non volatil, au moins un polymère liposoluble non siliconé et au moins un agent dispersant hydrocarboné qui présente les paramètres de solubilité δd et δa satisfaisant aux conditions suivantes $16,2 \leq \delta_d \leq 20$ $(J/cm^3)^{1/2}$ et $9,1 \leq \delta_a \leq 20$ $(J/cm^3)^{1/2}$, ledit agent dispersant comportant des atomes de carbone et d'hydrogène et une ou plusieurs fonctions choisies parmi les fonctions hydroxyle, ester, éther, carboxylique ou amide, ledit composé siliconé non rotatif étant une huile cosmétique, ledit agent dispersant hydrocarbone représentant de 0,5 à 40% du poids total de la composition, et ladite composition comprenant moins de 10% et huile volatile par rapport au poids total de la composition.

**33.** Procédé selon la revendication précédente, **caractérisé en ce que** l'agent dispersant hydrocarboné est choisi parmi:

- les alcools gras modifiés éther et en particulier les produits d'addition de l'oxyde d'éthytène et /ou de l'oxyde de propylène avec i) un alcool gras linéaire ou ramifié ou avec ii) un alkylphénol,
- les esters, de préférence les mono et diesters, résultant de la réaction d'acides gras avec des produits d'addition de l'oxyde d'éthylène et du glycérol ou avec des produits d'addition de l'oxyde d'éthylène et du polyglycérol,
- les esters résultant de la réaction d'au moins un acide gras avec au moins un produit d'addition de l'oxyde d'éthylène et du glycérol ou avec au moins un produit d'addition de l'oxyde d'éthylène et du polyglycérol,
- les esters résultant de la réaction du glycérol ou du polyglycérol avec au moins un produit d'addition de l'oxyde d'éthylène et d'un acide gras saturé ou insaturé,
- les esters partiels résultant de la réaction d'au moins un acide gras linéaire ou ramifié, saturé ou insaturé, d'acide ricinoléique ou d'acide 12-hydroxystéarique, avec au moins un polyol tel que le glycérol, le polyglycérol, le pentaérythritol, les alcools saccharidiques tels que le sorbitol, et en particulier les esters de polyglycérol.
- les esters résultant de la réaction du sorbitan avec au moins un acide gras linéaire ou ramifié, saturé ou insaturé,
- les esters de sorbitan modifiés éther, et en particulier tes esters résultant iii) de la réaction du sorbitan avec au moins un produit d'addition de l'oxyde d'éthylène et d'un acide gras saturé ou insaturé ou iv) de la réaction d'au moins un acide gras saturé ou insaturé avec au moins un produit d'addition de l'oxyde d'éthylène et du sorbitan,
- les produits d'addition de l'oxyde d'éthylène avec l'huile de ricin et / ou l'huile de ricin hydrogénée,
- les phosphate trialkylés et les mono, di et triphosphate alkylés,

et leurs mélanges.

**34.** Procédé selon la revendication 32 ou 33 **caractérisé en ce que** le composé siliconé non volatil est choisi parmi les polydiméthylsiloxanes non volatils ; les polydiméthylsiloxanes comportant des groupements alkyle, alcoxy ou phényle, pendant ou en bout de chaîne siliconée, groupements ayant de 2 à 24 atomes de carbone ; les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates; les silicones fluorées comportant un groupement fluoré pendant ou en bout de chaîne siliconée ayant de 1 à 12 atomes de carbone dont tout ou partie des hydrogènes est substitué par des atomes de fluor ; les résines de silicone, les gommes de silicone et leurs mélanges.

**35.** Procédé selon l'une des revendications 32 à 34, **caractérisé en ce que** le polymère liposoluble non siliconé est choisi parmi :

- les homopolymères d'oléfines non cristallins ramifiés, les copolymères d'oléfines non ramifiés, les homopolymères et copolymères de diènes hydrogénés non cristallins, les oligomères linéaires ou ramifiés, homo ou copolymères de (méth)acrylates d'alkyles ayant de préférence un groupement alkyle en $C_8$-$C_{30}$ et une masse molaire inférieure ou égale à 10000g/mol, les oligomères homo et copolymères de (méth)acrylates d'alkyles perfluorés ayant une masse molaire inférieure ou égale à 10000 g/mol, et mieux inférieure ou égale à 8000 g/mol, les oligomères homo et copolymères d'esters vinyliques ayant des groupements alkyles en $C_8$-$C_{30}$ et une masse molaire inférieure ou égale à 10000 g/mol, et mieux inférieure ou égale à 8000 g/mol, les oligomères homo et copolymères de vinyléthers ayant des groupements alkyles en $C_8$-$C_{30}$, ayant une masse molaire

inférieure ou égale à 10000 g/mol, et mieux inférieure ou égale à 8000 g/mol,
- les polyéthers liposolubles résultant de la polyétherification entre un ou plusieurs diols en $C_2$-$C_{100}$, de préférence en $C_2$-$C_{50}$.
- les polyesters non réticulés résultant de la polycondensation entre un diacide ou un polyacide carboxylique linéaire ou ramifié en $C_4$-$C_{50}$ et un diol ou un polyol en $C_2$-$C_{50}$,

et leurs mélanges.

**36.** Utilisation de l'association d'au moins un composé siliconé non volatil, d'au moins un polymère liposoluble non siliconé et d'au moins un agent dispersant hydrocarboné qui présente les paramètres de solubilité δd et δa satisfaisant aux conditions suivantes : $16{,}2 \leq \delta_d \leq 20\ (J/cm^3)^{1/2}$ et $9{,}1 \leq \delta_a \leq 20\ (J/cm^3)^{1/2}$ dans une composition cosmétique anhydre de bonne tenue, notamment de la couleur, brillante, confortable et /ou non migrante, ledit agent dispersant comportant des atomes de carbone et d'hydrogène et une ou plusieurs fonctions choisies parmi les fonctions hydroxyle, ester, éther, carboxylique ou amide, ledit composé siliconé non rotatif étant une huile cosmétique, ledit agent dispersant hydrocarbone représentant de 0,5 à 40% du poids total de la composition, et ladite composition comprenant moins de 10% et huile volatile par rapport au poids total de la composition.

**37.** Utilisation de l'association d'au moins un composé siliconé non volatil, d'au moins un polymère liposoluble non siliconé et d'au moins un agent dispersant hydrocarboné qui présente les paramètres de solubilité δd et δa satisfaisant aux conditions suivantes : $16{,}2 \leq \delta_d \leq 20\ (J/cm^3)^{1/2}$ et $9{,}1 \leq \delta_a \leq 20\ (J/cm^3)^{1/2}$, ledit agent dispersant comportant des atomes de carbone et d'hydrogène et une ou plusieurs fonctions choisies parmi les fonctions hydroxyle, ester, éther, carboxylique ou amide, dans une composition cosmétique anydre, comme agent pour conférer à ladite composition des propriétés de tenue, notamment de la couleur, de brillance, de confort et/ou de non-migration, ledit composé siliconé non rotatif étant une huile cosmétique, ledit agent dispersant hydrocarbone représentant de 0,5 à 40% du poids total de la composition, et ladite composition comprenant moins de 10% et huile volatile par rapport au poids total de la composition.

**38.** Utilisation selon la revendication 36 ou 37 **caractérisée en ce que** l'agent dispersant hydrocarboné est choisi parmi :

- les alcools gras modifiés éther et en particulier les produits d'addition de l'oxyde d'éthylène et /ou de l'oxyde de propylène avec i) un alcool gras linéaire ou ramifié ou avec ii) un alkylphénol,
- les esters résultant de la réaction d'au moins un acide gras avec au moins un produit d'addition de l'oxyde d'éthylène et du glycérol ou avec au moins un produit d'addition de l'oxyde d'éthylène et du polyglycérol,
- les esters résultant de la réaction du glycérol ou du polyglycérol avec au moins un produit d'addition de l'oxyde d'éthylène et d'un acide gras saturé ou insaturé,
- les esters partiels résultant de la réaction d'au moins un acide gras linéaire ou ramifié, saturé ou insaturé, d'acide ricinoléique ou d'acide 12-hydroxystéarique, avec au moins un polyol tel que le glycérol, le polyglycérol, le pentaérythritol, les alcools saccharidiques tels que le sorbitol, et en particulier les esters de polyglycérol,
- les esters résultant de la réaction du sorbitan avec au moins un acide gras linéaire ou ramifié, saturé ou insaturé,
- les esters de sorbitan modifiés éther, et en particulier les esters résultant iii) de la réaction du sorbitan avec au moins un produit d'addition de l'oxyde d'éthylène et d'un acide gras saturé ou insaturé ou iv) de la réaction d'au moins un acide gras saturé ou insaturé avec au moins un produit d'addition de l'oxyde d'éthylène et du sorbitan,
- les produits d'addition de l'oxyde d'éthylène avec l'huile de ricin et / ou l'huile de ricin hydrogénée,
- les phosphate trialkylés et les mono, di et triphosphate alkylés;

et leurs mélanges.

**39.** Utilisation selon l'une des revendications 36 à 38, **caractérisée en ce que** le composé siliconé non volatil est choisi parmi les polydiméthylsiloxanes non volatils ; les polydiméthylsiloxanes comportant des groupements alkyle, alcoxy ou phényle, pendant ou en bout de chaîne siliconée, groupements ayant de 2 à 24 atomes de carbone ; les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ; les silicones fluorées comportant un groupement fluoré pendant ou en bout de chaîne siliconée ayant de 1 à 12 atomes de carbone dont tout ou partie des hydrogènes est substitué par des atomes de fluor ; les résines de silicone; les gommes de silicone ; et leurs mélanges.

**40.** Utilisation selon l'une des revendications 36 à 40, **caractérisée en ce que** le polymère liposoluble non siliconéest

choisi parmi :

- les homopolymères d'oléfines non cristallins ramifiés, les copolymères d'oléfines non ramifiés, les homopolymères et copolymères de diènes hydrogénés non cristallins, les oligomères linéaires ou ramifiés, homo ou copolymères de (méth)acrytates d'alkyles ayant de préférence un groupement alkyle en $C_8$-$C_{30}$ et une masse molaire inférieure ou égale à 10000g/mol, les oligomères homo et copolymères de (méth)acrylates d'alkyles perfluorés ayant une masse molaire inférieure ou égale à 10000 g/mol, et mieux inférieure ou égale à 8000 g/mol, les oligomères homo et copolymères d'esters vinyliques ayant des groupements alkyles en $C_8$-$C_{30}$ et une masse molaire inférieure ou égale à 10000 g/mol, et mieux inférieure ou égale à 8000 g/mol, les oligomères homo et copolymères de vinyléthers ayant des groupements alkyles en $C_8$-$C_{30}$, ayant une masse molaire, inférieure ou égale à 10000 g/mol, et mieux inférieure ou égale à 8000 g/mol,
- les polyéthers liposolubles résultant de la polyétherification entre un ou plusieurs diols en $C_2$-$C_{100}$, de préférence en $C_2$-$C_{50}$,
- les polyesters non réticulés résultant de la polycondensation entre un diacide ou un polyacide carboxylique linéaire ou ramifié en $C_4$-$C_{50}$ et un diol ou un polyol en $C_2$-$C_{50}$, et leurs mélanges.

## Claims

1. Anhydrous composition for caring for or making up keratin materials, comprising a cosmetically acceptable medium containing

   - at least one non-volatile silicone compound, the said non-volatile silicone compound being a cosmetic oil,
   - at least one non-silicone liposoluble polymer, and
   - at least one hydrocarbon-based dispersant with solubility parameters δd and δa that satisfy the following conditions: $16.2 \leq \delta_d \leq 20$ $(J/cm^3)^{1/2}$ and $9.1 \leq \delta_a \leq 20$ $(J/cm^3)^{1/2}$, the said dispersant comprising carbon and hydrogen atoms and one or more functions chosen from hydroxyl, ester, ether, carboxylic and amide functions, the said hydrocarbon-based dispersant representing from 0.5% to 40% of the total weight of the composition,

   and the said composition comprising less than 10% volatile oil relative to the total weight of the composition.

2. Composition according to Claim 1, **characterized in that** it contains less than 5% and better still less than 2% volatile oil relative to the total weight of the composition.

3. Composition according to either of the preceding claims, **characterized in that** the hydrocarbon-based dispersant has solubility parameters δd and δa that satisfy the following conditions: $16.3 \leq \delta_d \leq 19$ $(J/cm^3)^{1/2}$ and $10 \leq \delta_a \leq 18.1$ $(J/cm^3)^{1/2}$.

4. Composition according to one of the preceding claims, **characterized in that** the hydrocarbon-based dispersant has solubility parameters δd and δa that satisfy the following conditions: $16.9 \leq \delta_d \leq 18$ $(J/cm^3)^{1/2}$ and $13 \leq \delta_a \leq 14.5$ $(J/cm^3)^{1/2}$.

5. Composition according to one of the preceding claims, **characterized in that** the hydrocarbon-based dispersant is fluid at room temperature (25°C) and/or has a refractive index ≥ 1.45 at 20°C.

6. Composition according to one of the preceding claims, **characterized in that** the hydrocarbon-based dispersant has a chemical structure comprising at least one polar group chosen from -COOH; -OH; ethylene oxide: - ($O$-$CH_2$-$CH_2$-) ; propylene oxide

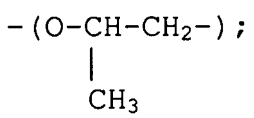

   -$PO_4$; NHR; $NR_1R_2$ with $R_1$ and $R_2$ optionally forming a ring and representing a linear or branched $C_1$-$C_{20}$ alkyl or alkoxy radical or

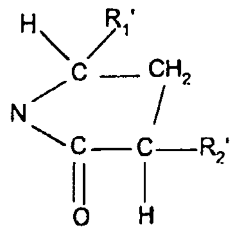

with $R_1$' and $R_2$' which may be equal to H or to a linear or branched $C_1$-$C_{20}$ alkyl or alkoxy chain.

**7.** Composition according to one of the preceding claims, **characterized in that** the hydrocarbon-based dispersant is chosen from:

- ether-modified fatty alcohols and in particular adducts of ethylene oxide and/or propylene oxide with i) a linear or branched fatty alcohol or with ii) an alkylphenol,
- esters resulting from the reaction of at least one fatty acid with at least one adduct of ethylene oxide and of glycerol or with at least one adduct of ethylene oxide and of polyglycerol,
- esters resulting from the reaction of glycerol or polyglycerol with at least one adduct of ethylene oxide and of a saturated or unsaturated fatty acid,
- partial esters resulting from the reaction of at least one linear or branched, saturated or unsaturated fatty acid, ricinoleic acid or 12-hydroxystearic acid with at least one polyol such as glycerol, polyglycerol, pentaerythritol or saccharide alcohols such as sorbitol, and in particular polyglycerol esters,
- esters resulting from the reaction of sorbitan with at least one linear or branched, saturated or unsaturated fatty acid,
- ether-modified sorbitan esters, and in particular esters resulting iii) from the reaction of sorbitan with at least one adduct of ethylene oxide and of a saturated or unsaturated fatty acid or iv) from the reaction of at least one saturated or unsaturated fatty acid with at least one adduct of ethylene oxide and of sorbitan,
- adducts of ethylene oxide with castor oil and/or hydrogenated castor oil,
- trialkyl phosphates and alkyl monophosphates, diphosphates and triphosphates,

and mixtures thereof.

**8.** Composition according to one of the preceding claims, **characterized in that** the hydrocarbon-based dispersant is chosen from monoesters, diesters and esters resulting from a partial esterification.

**9.** Composition according to one of the preceding claims, **characterized in that** the hydrocarbon-based dispersant is chosen from:

- adducts of 2 to 30 mol of ethylene oxide and/or of 0 to 5 mol of propylene oxide with i) a linear or branched $C_8$-$C_{40}$ and better still $C_8$-$C_{22}$ fatty alcohol or with ii) an alkylphenol,
- esters resulting from the reaction of at least one $C_8$-$C_{40}$ and better still $C_8$-$C_{22}$ fatty acid with at least one adduct of 1 to 30 mol of ethylene oxide and of glycerol or with at least one adduct of 1 to 30 mol of ethylene oxide and of polyglycerol,
- esters resulting from the reaction of glycerol or polyglycerol with at least one adduct of 2 to 30 mol of ethylene oxide and of a saturated or unsaturated $C_8$-$C_{40}$ and better still $C_8$-$C_{22}$ fatty acid,
- partial esters resulting from the reaction of at least one linear or branched, saturated or unsaturated $C_8$-$C_{40}$ and better still $C_8$-$C_{22}$ fatty acid, ricinoleic acid or 12-hydroxystearic acid with glycerol, polyglycerol, pentaerythritol or sorbitol,
- esters resulting from the reaction of sorbitan with at least one linear or branched, saturated or unsaturated $C_8$-$C_{40}$ and better still $C_8$-$C_{22}$ fatty acid,
- esters resulting iii) from the reaction of sorbitan with at least one adduct of 2 to 30 mol of ethylene oxide and of a saturated or unsaturated $C_8$-$C_{40}$ and better still $C_8$-$C_{22}$ fatty acid or iv) from the reaction of at least one saturated or unsaturated $C_8$-$C_{40}$ and better still $C_8$-$C_{22}$ fatty acid with at least one adduct of 2 to 30 mol of ethylene oxide and of sorbitan,
- adducts of 2 to 60 mol of ethylene oxide with castor oil and/or hydrogenated castor oil,
- trialkyl phosphates and alkyl monophosphates, diphosphates and triphosphates, and mixtures thereof.

**10.** Composition according to one of the preceding claims, **characterized in that** the hydrocarbon-based dispersant is chosen from: myristyl alcohol oxyethylenated with 15 EO, polyglyceryl-2 monoisostearate oxyethylenated with 5 EO, polyglyceryl-3 diisostearate, glyceryl monoisostearate, polyglyceryl-2 monoisostearate, polyglyceryl-3 isostearate, polyglyceryl-4 isostearate, polyglyceryl-6 monoisostearate, polyglyceryl-10 monoisostearate, polyglyceryl-2 monooleate, sorbitan isostearate, sorbitan monooleate and sorbitan monooleate oxyethylenated with 5 EO, and mixtures thereof.

**11.** Composition according to one of the preceding claims, **characterized in that** the hydrocarbon-based dispersant is chosen from partial esters of polyglycerol and of isostearic acid, partial esters of polyglycerol and of oleic acid, and partial esters of sorbitan and of oleic acid, and mixtures thereof.

**12.** Composition according to one of the preceding claims, **characterized in that** the hydrocarbon-based dispersant is chosen from polyglyceryl-3 diisostearate, polyglyceryl-2 monoisostearate, polyglyceryl-2 monooleate and sorbitan monooleate, and mixtures thereof.

**13.** Composition according to one of the preceding claims, **characterized in that** the hydrocarbon-based dispersant represents from 3% to 20% and better still from 5% to 15% of the total weight of the composition.

**14.** Composition according to the preceding claim, in which the non-volatile silicone compound has a viscosity ranging from 5 to 1 000 000 cSt, preferably ranging from 10 to 500 000 cSt and better still from 10 to 5000 cSt, at 25°C.

**15.** Composition according to one of the preceding claims, in which the non-volatile silicone compound is chosen from non-volatile polydimethylsiloxanes; polydimethylsiloxanes comprising alkyl, alkoxy or phenyl groups, which are pendent or at the end of a silicone chain, these groups containing from 2 to 24 carbon atoms; phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates; fluoro silicones comprising a fluoro group that is pendent or at the end of a silicone chain, containing from 1 to 12 carbon atoms, all or some of the hydrogens of which are substituted with fluorine atoms; silicone gums; and mixtures thereof.

**16.** Composition according to one of the preceding claims, **characterized in that** the non-volatile silicone compound represents from 0.5% to 90%, preferably from 5% to 60% and better still from 10% to 50% of the total weight of the composition.

**17.** Composition according to one of the preceding claims, **characterized in that** the non-silicone liposoluble polymer has a molecular mass ranging from 400 to 500 000 g/mol and preferably from 500 to 100 000 g/mol.

**18.** Composition according to one of the preceding claims, **characterized in that** the non-silicone liposoluble polymer is chosen from vinyl polymers, liposoluble polyethers and non-crosslinked polyesters, and mixtures thereof.

**19.** Composition according to one of the preceding claims, **characterized in that** the non-silicone liposoluble polymer is chosen from:

- branched non-crystalline olefin homopolymers, non-branched olefin copolymers, non-crystalline hydrogenated diene homopolymers and copolymers, linear or branched oligomers, alkyl (meth)acrylate homopolymers or copolymers preferably containing a $C_8$-$C_{30}$ alkyl group and having a molar mass of less than or equal to 10 000 g/mol, perfluoroalkyl (meth)acrylate homopolymer and copolymer oligomers with a molar mass of less than or equal to 10 000 g/mol and better still less than or equal to 8000 g/mol, vinyl ester homopolymer and copolymer oligomers containing $C_8$-$C_{30}$ alkyl groups and having a molar mass of less than or equal to 10 000 g/mol and better still less than or equal to 8000 g/mol, vinyl ether homopolymer and copolymer oligomers containing $C_8$-$C_{30}$ alkyl groups and having a molar mass of less than or equal to 10 000 g/mol and better still less than or equal to 8000 g/mol,
- liposoluble polyethers resulting from poly-etherification between one or more $C_2$-$C_{100}$ and preferably $C_2$-$C_{50}$ diols,
- non-crosslinked polyesters resulting from polycondensation between a linear or branched $C_4$-$C_{50}$ dicarboxylic or polycarboxylic acid and a $C_2$-$C_{50}$ diol or polyol, and mixtures thereof.

**20.** Composition according to one of the preceding claims, **characterized in that** the liposoluble polymer is chosen from polybutylenes, hydrogenated polyisobutylenes, polydecenes, hydrogenated polydecenes, vinylpyrrolidone (VP)/olefin copolymers with a carbon number ranging from 8 to 30 and preferably from 10 to 30, hydrogenated polybutadiene, hydrogenated polyisoprene, polyvinyl laurate, vinyl acetate/allyl stearate copolymer and PEG-45/dodecyl glycol copolymer, and mixtures thereof.

**21.** Composition according to one of the preceding claims, **characterized in that** the liposoluble polymer represents from 1% to 98.5%, preferably from 2% to 85%, better still from 5% to 70% and even better still from 5% to 60% of the total weight of the composition.

**22.** Composition according to one of the preceding claims, **characterized in that** it contains at least one dyestuff.

**23.** Composition according to the preceding claim, **characterized in that** the dyestuff is chosen from dyes that are soluble or dispersible in the composition, pigments and nacres, and mixtures thereof.

**24.** Composition according to Claim 22 or 23, **characterized in that** the dyestuff represents from 0.5% to 50%, preferably from 2% to 40% and better still from 5% to 30% relative to the total weight of the composition.

**25.** Composition according to one of the preceding claims, **characterized in that** it contains a particulate phase.

**26.** Composition according to one of the preceding claims, **characterized in that** it also contains at least one wax.

**27.** Composition according to the preceding claim, **characterized in that** the wax represents from 0.01% to 50%, preferably from 2% to 40% and better still from 5% to 30% of the total weight of the composition.

**28.** Composition according to one of the preceding claims, **characterized in that** it also contains at least one additive chosen from antioxidants, preserving agents, neutralizers, lipophilic gelling agents or liquid non-aqueous compounds, dispersants and cosmetic active agents, and mixtures thereof.

**29.** Composition according to one of the preceding claims, **characterized in that** it contains at least one cosmetic active agent chosen from vitamins (A, E, C, $B_3$ and F), provitamins, calmatives, plant extracts or essential oils, protective or restructuring agents, refreshing active agents, emollients, moisturizers, anti-wrinkle active agents and essential fatty acids, and mixtures thereof.

**30.** Composition according to one of the preceding claims, **characterized in that** it is in the form of a cast or compacted product.

**31.** Composition according to one of the preceding claims, **characterized in that** it is in the form of a lipstick or a lip gloss.

**32.** Cosmetic process for giving a film of anhydrous cosmetic composition properties of colour-fastness, gloss, comfort and/or migration resistance, which consists in introducing into the said composition at least one non-volatile silicone compound, at least one non-silicone liposoluble polymer and at least one hydrocarbon-based dispersant with solubility parameters δd and δa that satisfy the following conditions: $16.2 \leq \delta_d \leq 20\ (J/cm^3)^{1/2}$ and $9.1 \leq \delta_a \leq 20\ (J/cm^3)^{1/2}$, the said dispersant comprising carbon and hydrogen atoms and one or more functions chosen from hydroxyl, ester, ether, carboxylic and amide functions, the said non-volatile silicone compound being a cosmetic oil, the said hydrocarbon-based dispersant representing from 0.5% to 40% of the total weight of the composition, and the said composition comprising less than 10% volatile oil relative to the total weight of the composition.

**33.** Process according to the preceding claim, **characterized in that** the hydrocarbon-based dispersant is chosen from:

- ether-modified fatty alcohols and in particular adducts of ethylene oxide and/or propylene oxide with i) a linear or branched fatty alcohol or with ii) an alkylphenol,
- esters, preferably monoesters or diesters, resulting from the reaction of fatty acids with adducts of ethylene oxide and of glycerol or with adducts of ethylene oxide and of polyglycerol,
- esters resulting from the reaction of at least one fatty acid with at least one adduct of ethylene oxide and of glycerol or with at least one adduct of ethylene oxide and of polyglycerol,
- esters resulting from the reaction of glycerol or polyglycerol with at least one adduct of ethylene oxide and of a saturated or unsaturated fatty acid,
- partial esters resulting from the reaction of at least one linear or branched, saturated or unsaturated fatty acid, ricinoleic acid or 12-hydroxystearic acid with at least one polyol such as glycerol, polyglycerol, pentaerythritol

or saccharide alcohols such as sorbitol, and in particular polyglycerol esters,
- esters resulting from the reaction of sorbitan with at least one linear or branched, saturated or unsaturated fatty acid,
- ether-modified sorbitan esters, and in particular esters resulting iii) from the reaction of sorbitan with at least one adduct of ethylene oxide and of a saturated or unsaturated fatty acid or iv) from the reaction of at least one saturated or unsaturated fatty acid with at least one adduct of ethylene oxide and of sorbitan,
- adducts of ethylene oxide with castor oil and/or hydrogenated castor oil,
- trialkyl phosphates and alkyl monophosphates, diphosphates and triphosphates, and mixtures thereof.

**34.** Process according to Claim 32 or 33, **characterized in that** the non-volatile silicone compound is chosen from non-volatile polydimethylsiloxanes; polydimethylsiloxanes comprising alkyl, alkoxy or phenyl groups, which are pendent or at the end of a silicone chain, these groups containing from 2 to 24 carbon atoms; phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates; fluoro silicones comprising a fluoro group that is pendent or at the end of a silicone chain, containing from 1 to 12 carbon atoms, all or some of the hydrogens of which are substituted with fluorine atoms; silicone resins; silicone gums; and mixtures thereof.

**35.** Process according to one of Claims 32 to 34, **characterized in that** the non-silicone liposoluble polymer is chosen from:

- branched non-crystalline olefin homopolymers, non-branched olefin copolymers, non-crystalline hydrogenated diene homopolymers and copolymers, linear or branched oligomers, alkyl (meth)acrylate homopolymers or copolymers preferably containing a $C_8$-$C_{30}$ alkyl group and having a molar mass of less than or equal to 10 000 g/mol, perfluoroalkyl (meth)acrylate homopolymer and copolymer oligomers with a molar mass of less than or equal to 10 000 g/mol and better still less than or equal to 8000 g/mol, vinyl ester homopolymer and copolymer oligomers containing $C_8$-$C_{30}$ alkyl groups and having a molar mass of less than or equal to 10 000 g/mol and better still less than or equal to 8000 g/mol, vinyl ether homopolymer and copolymer oligomers containing $C_8$-$C_{30}$ alkyl groups and having a molar mass of less than or equal to 10 000 g/mol and better still less than or equal to 8000 g/mol,
- liposoluble polyethers resulting from poly-etherification between one or more $C_2$-$C_{100}$ and preferably $C_2$-$C_{50}$ diols,
- non-crosslinked polyesters resulting from polycondensation between a linear or branched $C_4$-$C_{50}$ dicarboxylic or polycarboxylic acid and a $C_2$-$C_{50}$ diol or polyol, and mixtures thereof.

**36.** Use of the combination of at least one non-volatile silicone compound, at least one non-silicone liposoluble polymer and at least one hydrocarbon-based dispersant with solubility parameters δd and δa that satisfy the following conditions: $16.2 \le \delta_d \le 20$ $(J/cm^3)^{1/2}$ and $9.1 \le \delta_a \le 20$ $(J/cm^3)^{1/2}$, in a glossy, comfortable and/or migration-resistant anhydrous cosmetic composition with good fastness, especially colour fastness, the said dispersant comprising carbon and hydrogen atoms and one or more functions chosen from hydroxyl, ester, ether, carboxylic and amide functions, the said non-volatile silicone compound being a cosmetic oil, the said hydrocarbon-based dispersant representing from 0.5% to 40% of the total weight of the composition,
and the said composition comprising less than 10% volatile oil relative to the total weight of the composition.

**37.** Use of the combination of at least one non-volatile silicone compound, at least one non-silicone liposoluble polymer and at least one hydrocarbon-based dispersant with solubility parameters δd and δa that satisfy the following conditions: $16.2 \le \delta_d \le 20$ $(J/cm^3)^{1/2}$ and $9.1 \le \delta_a \le 20$ $(J/cm^3)^{1/2}$, the said dispersant comprising carbon and hydrogen atoms and one or more functions chosen from hydroxyl, ester, ether, carboxylic and amide functions, in an anhydrous cosmetic composition, as an agent for giving the said composition fastness, especially colour-fastness, gloss, comfort and/or migration-resistance properties, the said non-volatile silicone compound being a cosmetic oil, the said hydrocarbon-based dispersant representing from 0.5% to 40% of the total weight of the composition,
and the said composition comprising less than 10% volatile oil relative to the total weight of the composition.

**38.** Use according to Claim 36 or 37, **characterized in that** the hydrocarbon-based dispersant is chosen from:

- ether-modified fatty alcohols and in particular adducts of ethylene oxide and/or propylene oxide with i) a linear or branched fatty alcohol or with ii) an alkylphenol,
- esters resulting from the reaction of at least one fatty acid with at least one adduct of ethylene oxide and of glycerol or with at least one adduct of ethylene oxide and of polyglycerol,

- esters resulting from the reaction of glycerol or polyglycerol with at least one adduct of ethylene oxide and of a saturated or unsaturated fatty acid,
- partial esters resulting from the reaction of at least one linear or branched, saturated or unsaturated fatty acid, ricinoleic acid or 12-hydroxystearic acid with at least one polyol such as glycerol, polyglycerol, pentaerythritol or saccharide alcohols such as sorbitol, and in particular polyglycerol esters,
- esters resulting from the reaction of sorbitan with at least one linear or branched, saturated or unsaturated fatty acid,
- ether-modified sorbitan esters, and in particular esters resulting iii) from the reaction of sorbitan with at least one adduct of ethylene oxide and of a saturated or unsaturated fatty acid or iv) from the reaction of at least one saturated or unsaturated fatty acid with at least one adduct of ethylene oxide and of sorbitan,
- adducts of ethylene oxide with castor oil and/or hydrogenated castor oil,
- trialkyl phosphates and alkyl monophosphates, diphosphates and triphosphates, and mixtures thereof.

**39.** Use according to one of Claims 36 to 38, **characterized in that** the non-volatile silicone compound is chosen from non-volatile polydimethylsiloxanes; polydimethylsiloxanes comprising alkyl, alkoxy or phenyl groups, which are pendent or at the end of a silicone chain, these groups containing from 2 to 24 carbon atoms; phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates; fluoro silicones comprising a fluoro group that is pendent or at the end of a silicone chain, containing from 1 to 12 carbon atoms, all or some of the hydrogens of which are substituted with fluorine atoms; silicone resins; silicone gums; and mixtures thereof.

**40.** Use according to one of Claims 36 to 39, **characterized in that** the non-silicone liposoluble polymer is chosen from:

- branched non-crystalline olefin homopolymers, non-branched olefin copolymers, non-crystalline hydrogenated diene homopolymers and copolymers, linear or branched oligomers, alkyl (meth)acrylate homopolymers or copolymers preferably containing a $C_8$-$C_{30}$ alkyl group and having a molar mass of less than or equal to 10 000 g/mol, perfluoroalkyl (meth)acrylate homopolymer and copolymer oligomers with a molar mass of less than or equal to 10 000 g/mol and better still less than or equal to 8000 g/mol, vinyl ester homopolymer and copolymer oligomers containing $C_8$-$C_{30}$ alkyl groups and having a molar mass of less than or equal to 10 000 g/mol and better still less than or equal to 8000 g/mol, vinyl ether homopolymer and copolymer oligomers containing $C_8$-$C_{30}$ alkyl groups and having a molar mass of less than or equal to 10 000 g/mol and better still less than or equal to 8000 g/mol,
- liposoluble polyethers resulting from poly-etherification between one or more $C_2$-$C_{100}$ and preferably $C_2$-$C_{50}$ diols,
- non-crosslinked polyesters resulting from polycondensation between a linear or branched $C_4$-$C_{50}$ dicarboxylic or polycarboxylic acid and a $C_2$-$C_{50}$ diol or polyol,

and mixtures thereof.

**Patentansprüche**

**1.** Wasserfreie Zusammensetzung zur Pflege oder zum Schminken von Keratinsubstanzen, die in einem kosmetisch akzeptablen Medium enthält:

- mindestens eine nichtflüchtige Siliconverbindung, wobei die nichtflüchtige Siliconverbindung ein kosmetisches Öl ist,
- mindestens ein nicht siliconiertes, fettlösliches Polymer, und
- mindestens ein Dispergiermittel auf Kohlenwasserstoffbasis, das Solubilitätsparameter δd und δa aufweist, die die folgenden Bedingungen erfüllen: $16{,}2 \leq \delta_d \leq 20\ (J/cm^3)^{1/2}$ und $9{,}1 \leq \delta_a \leq 20\ (J/cm^3)^{1/2}$, wobei das Dispergiermittel Kohlenstoffatome und Wasserstoffatome und eine oder mehrere Funktionen enthält, die unter den Funktionen Hydroxy, Ester, Ether, Carboxy und Amid ausgewählt sind,

wobei das Dispergiermittel auf Kohlenwasserstoffbasis 0,5 bis 40 % des Gesamtgewichts der Zusammensetzung ausmacht und wobei die Zusammensetzung weniger als 10 % flüchtiges Öl, bezogen auf das Gesamtgewicht der Zusammensetzung, enthält.

**2.** Zusammensetzung nach Anspruch 1, **dadurch gekennzeichnet, dass** sie weniger als 5 % flüchtiges Öl, bezogen

auf das Gesamtgewicht der Zusammensetzung, und besser weniger als 2 % enthält.

**3.** Zusammensetzung nach einem der vorhergehenden Ansprüche, **dadurch gekennzeichnet, dass** das Dispergiermittel auf Kohlenwasserstoffbasis Solubilitätsparameter δd und δa aufweist, die die folgenden Bedingungen erfüllen: $16{,}3 \leq \delta_d \leq 19\ (J/cm^3)^{1/2}$ und $10 \leq \delta_a \leq 18{,}1\ (J/cm^3)^{1/2}$.

**4.** Zusammensetzung nach einem der vorhergehenden Ansprüche, **dadurch gekennzeichnet, dass** das Dispergiermittel auf Kohlenwasserstoffbasis Solubilitätsparameter δd und δa aufweist, die die folgenden Bedingungen erfüllen: $16{,}9 \leq \delta_d \leq 18\ (J/cm^3)^{1/2}$ und $13 \leq \delta_a \leq 14{,}5\ (J/cm^3)^{1/2}$.

**5.** Zusammensetzung nach einem der vorhergehenden Ansprüche, **dadurch gekennzeichnet, dass** das Dispergiermittel auf Kohlenwasserstoffbasis bei Raumtemperatur (25 °C) fluide ist und/oder bei 20 °C eine Brechzahl ≥ 1,45 aufweist.

**6.** Zusammensetzung nach einem der vorhergehenden Ansprüche, **dadurch gekennzeichnet, dass** das Dispergiermittel auf Kohlenwasserstoffbasis eine chemische Struktur aufweist, die mindestens eine polare Gruppe enthält, die unter den folgenden Gruppen ausgewählt ist: -COOH; -OH; Ethylenoxid: $-(O-CH_2-CH_2-)$; Propylenoxid

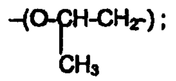

$-PO_4$; NHR; $NR_1R_2$, wobei $R_1$ und $R_2$ gegebenenfalls einen Ring bilden und eine geradkettige oder verzweigte Alkyl- oder Alkoxygruppe mit 1 bis 20 Kohlenstoffatomen bedeuten oder

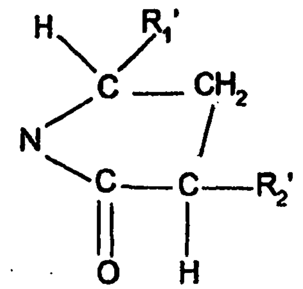

wobei $R_1'$ und $R_2'$ H oder eine geradkettige oder verzweigte Alkyl- oder Alkoxygruppe mit 1 bis 20 Kohlenstoffatomen bedeuten können.

**7.** Zusammensetzung nach einem der vorhergehenden Ansprüche, **dadurch gekennzeichnet, dass** das Dispergiermittel auf Kohlenwasserstoffbasis ausgewählt ist unter:

- Ether-modifizierten Fettalkoholen und insbesondere Additionsprodukten von Ethylenoxid und/oder Propylenoxid mit i) einem geradkettigen oder verzweigten Fettalkohol oder ii) einem Alkylphenol,
- Estern, die bei der Reaktion mindestens einer Fettsäure mit mindestens einem Additionsprodukt von Ethylenoxid und Glycerin oder mit mindestens einem Additionsprodukt von Ethylenoxid und Polyglycerin gebildet werden,
- Estern, die bei der Reaktion von Glycerin oder Polyglycerin mit mindestens einem Additionsprodukt von Ethylenoxid und einer gesättigten oder ungesättigten Fettsäure gebildet werden,
- partiellen Estern, die bei der Reaktion mindestens einer geradkettigen oder verzweigten, gesättigten oder ungesättigten Fettsäure, Ricinolsäure oder 12-Hydroxystearinsäure mit mindestens einem Polyol wie Glycerin, Polyglycerin, Pentaerythrit, Saccharidalkoholen wie Sorbit gebildet werden, und insbesondere Polyglycerylestern,

- Estern, die bei der Reaktion von Sorbitan mit mindestens einer geradkettigen oder verzweigten, gesättigten oder ungesättigten Fettsäure gebildet werden,
- Ether-modifizierten Sorbitanestern und insbesondere Estern, die iii) bei der Reaktion von Sorbitan mit mindestens einem Additionsprodukt von Ethylenoxid und einer gesättigten oder ungesättigten Fettsäure gebildet werden, oder iv) die bei der Reaktion von mindestens einer gesättigten oder ungesättigten Fettsäure mit mindestens einem Additionsprodukt von Ethylenoxid und Sorbitan gebildet werden,
- Additionsprodukten von Ethylenoxid und Ricinusöl und/oder hydriertem Ricinusöl,
- Trialkylphosphaten und alkylierten Mono-, Di- und Triphosphaten,

und deren Gemischen.

**8.** Zusammensetzung nach einem der vorhergehenden Ansprüche, **dadurch gekennzeichnet, dass** das Dispergiermittel auf Kohlenwasserstoffbasis unter den Monoestern, Diestern und Estern, die bei einer partiellen Veresterung gebildet werden, ausgewählt ist.

**9.** Zusammensetzung nach einem der vorhergehenden Ansprüche, **dadurch gekennzeichnet, dass** das Dispergiermittel auf Kohlenwasserstoffbasis ausgewählt ist unter:

- Additionsprodukten von 2 bis 30 mol Ethylenoxid und/oder 0 bis 5 mol Propylenoxid mit i) einem geradkettigen oder verzweigten $C_{8-40}$-Fettalkohol oder besser einem $C_{8-22}$-Fettalkohl oder mit ii) einem Alkylphenol,
- Estern, die bei der Reaktion mindestens einer $C_{8-40}$-Fettsäure und besser $C_{8-22}$-Fettsäure mit mindestens einem Additionsprodukt von 1 bis 30 mol Ethylenoxid und Glycerin oder mit mindestens einem Additionsprodukt von 1 bis 30 mol Ethylenoxid und Polyglycerin gebildet werden,
- Estern, die bei der Reaktion von Glycerin oder Polyglycerin mit mindestens einem Additionsprodukt von 2 bis 30 mol Ethylenoxid und einer gesättigten oder ungesättigten $C_{8-40-}$Fettsäure und besser $C_{8-22}$-Fettsäure gebildet werden,
- partiellen Estern, die bei der Reaktion mindestens einer geradkettigen oder verzweigten, gesättigten oder ungesättigten Fettsäure mit 8 bis 40 Kohlenstoffatomen und besser 8 bis 22 Kohlenstoffatomen, Ricinolsäure oder 12-Hydroxystearinsäure mit Glycerin, Polyglycerin, Pentaerythrit oder Sorbit gebildet werden,
- Estern, die bei der Reaktion von Sorbitan mit mindestens einer geradkettigen oder verzweigten, gesättigten oder ungesättigten Fettsäure mit 8 bis 40 Kohlenstoffatomen und besser 8 bis 22 Kohlenstoffatomen gebildet werden,
- Estern, die iii) bei der Reaktion von Sorbitan mit mindestens einem Additionsprodukt von 2 bis 30 mol Ethylenoxid und einer gesättigten oder ungesättigten $C_{8-40}$-Fettsäure und besser $C_{8-22}$-Fettsäure oder iv) bei der Umsetzung von mindestens einer gesättigten oder ungesättigten $C_{8-40}$-Fettsäure und besser $C_{8-22}$-Fettsäure mit mindestens einem Additionsprodukt von 2 bis 30 mol Ethylenoxid und Sorbitan gebildet werden,
- Additionsprodukten von 2 bis 60 mol Ethylenoxid und Ricinusöl und/oder hydriertem Ricinusöl,
- trialkylierten Phosphaten und alkylierten Mono-, Di- und Triphosphaten,

und deren Gemischen.

**10.** Zusammensetzung nach einem der vorhergehenden Ansprüche, **dadurch gekennzeichnet, dass** das Dispergiermittel auf Kohlenwasserstoffbasis ausgewählt ist unter: mit 15 EO ethoxyliertem Myristylalkohol, mit 5 EO ethoxyliertem Diglycerylmonoisostearat, Triglyceryldiisostearat, Glycerylmonoisostearat, Diglycerylmonoisostearat, Triglycerylisostearat, Tetraglycerylisostearat, Hexaglycerylmonoisostearat, Decaglycerylmonoisostearat, Diglycerylmonooleat, Sorbitanisostearat, Sorbitanmonooleat, mit 5 EO ethoxyliertem Sorbitanmonooleat und deren Gemischen.

**11.** Zusammensetzung nach einem der vorhergehenden Ansprüche, **dadurch gekennzeichnet, dass** das Dispergiermittel auf Kohlenwasserstoffbasis unter den partiellen Estern von Polyglycerin und Isostearinsäure, partiellen Estern von Polyglycerin und Ölsäure, partiellen Estern von Sorbitan und Ölsäure und deren Gemischen ausgewählt ist.

**12.** Zusammensetzung nach einem der vorhergehenden Ansprüche, **dadurch gekennzeichnet, dass** das Dispergiermittel auf Kohlenwasserstoffbasis unter Triglyceryldiisostearat, Diglycerylmonoisostearat, Diglycerylmonooleat, Sorbitanmonooleat und deren Gemischen ausgewählt ist.

**13.** Zusammensetzung nach einem der vorhergehenden Ansprüche, **dadurch gekennzeichnet, dass** das Dispergiermittel auf Kohlenwasserstoffbasis 3 bis 20 % und besser 5 bis 15 % des Gesamtgewichts der Zusammensetzung

ausmacht.

**14.** Zusammensetzung nach dem vorhergehenden Anspruch, **dadurch gekennzeichnet, dass** die nichtflüchtige Siliconverbindung bei 25 °C eine Viskosität von 5 bis 1 000 000 cSt, vorzugsweise 10 bis 500 000 cSt und noch besser 10 bis 5 000 cSt aufweist.

**15.** Zusammensetzung nach einem der vorhergehenden Ansprüche, **dadurch gekennzeichnet, dass** die nichtflüchtige Siliconverbindung unter den nichtflüchtigen Polydimethylsiloxanen; Polydimethylsiloxanen, die als Seitenkette oder am Ende der Siliconkette Alkyl-, Alkoxy- oder Phenylgruppen aufweisen, wobei diese Gruppen 2 bis 24 Kohlenstoffatomen besitzen; Phenyltrimethiconen, Phenyldimethiconen, Phenyltrimethylsiloxydiphenylsiloxanen, Diphenyldimethiconen, Diphenylmethyldiphenyltrisiloxanen, 2-Phenylethyltrimethylsiloxysilicaten; fluorierten Siliconen, die als Seitenkette oder am Ende der Siliconkette eine fluorierte Gruppe mit 1 bis 12 Kohlenstoffatomen aufweisen, bei der alle oder ein Teil der Kohlenwasserstoffatome durch Fluoratome ersetzt sind; Silicongummis; und deren Gemischen ausgewählt ist.

**16.** Zusammensetzung nach einem der vorhergehenden Ansprüche, **dadurch gekennzeichnet, dass** die nichtflüchtige Siliconverbindung 0,5 bis 90 %, vorzugsweise 5 bis 60 % und besser 10 bis 50 % des Gesamtgewichts der Zusammensetzung ausmacht.

**17.** Zusammensetzung nach einem der vorhergehenden Ansprüche, **dadurch gekennzeichnet, dass** das nicht siliconhaltige, fettlösliche Polymer eine Molmasse von 400 bis 500 000 g/mol und vorzugsweise 500 bis 100 000 g/mol besitzt.

**18.** Zusammensetzung nach einem der vorhergehenden Ansprüche, **dadurch gekennzeichnet, dass** das nicht siliconhaltige, fettlösliche Polymer unter den Vinylpolymeren, fettlöslichen Polyethern, nicht vernetzten Polyestern und deren Gemischen ausgewählt ist.

**19.** Zusammensetzung nach einem der vorhergehenden Ansprüche, **dadurch gekennzeichnet, dass** das nicht siliconhaltige, fettlösliche Polymer ausgewählt ist unter:

- verzweigten, nicht kristallinen Homopolymeren von Olefinen, nicht verzweigten Copolymeren von Olefinen, nicht kristallinen, hydrierten Homopolymeren und Copolymeren von Dienen, geradkettigen oder verzweigten, oligomeren Homo- oder Copolymeren von Alkyl(meth)acrylaten, die vorzugsweise eine $C_{8-30}$-Alkylgruppe enthalten und eine Molmasse von höchstens 10 000 g/mol besitzen, oligomeren Homo- und Copolymeren von perfluorierten Alkyl(meth)acrylaten, die eine Molmasse von höchstens 10 000 g/mol und noch besser 8000 g/mol oder darunter besitzen, oligomeren Homo- und Copolymeren von Vinylestern, die $C_{8-30}$-Alkylgruppen aufweisen und eine Molmasse von höchstens 10 000 g/mol und noch besser 8000 g/mol oder darunter besitzen, oligomeren Homo- und Copolymeren von Vinylethern, die $C_{8-30}$-Alkylgruppen aufweisen und eine Molmasse von höchstens 10 000 g/mol und noch besser 8000 g/mol oder darunter besitzen,
- fettlöslichen Polyethern, die bei der mehrfachen Veretherung eines oder mehrerer $C_{2-100}$-Diole und vorzugsweise $C_{2-50}$-Diole gebildet werden,
- nicht vernetzten Polyestern, die bei der Polykondensation einer geradkettigen oder verzweigten Dicarbonsäure oder Polycarbonsäure mit 4 bis 50 Kohlenstoffatomen und eines Diols oder Polyols mit 2 bis 50 Kohlenstoffatomen gebildet werden,
- und deren Gemischen.

**20.** Zusammensetzung nach einem der vorhergehenden Ansprüche, **dadurch gekennzeichnet, dass** das fettlösliche Polymer ausgewählt ist unter: Polybutylenen, hydrierten Polyisobutylenen, Polydecenen, hydrierten Polydecenen, Copolymeren Vinylpyrrolidon (VP) / Olefine mit einer Kohlenstoffzahl von 8 bis 30 und vorzugsweise 10 bis 30, hydriertem Polybutadien, hydriertem Polyisopren, Polyvinyllaurat, dem Copolymer Vinylacetat/Allylstearat, dem Copolymer PEG-45/Dodecylglycol und deren Gemischen.

**21.** Zusammensetzung nach einem der vorhergehenden Ansprüche, **dadurch gekennzeichnet, dass** das fettlösliche Polymer 1 bis 98,5 %, vorzugsweise 2 bis 85 %, besser 5 bis 70 % und noch besser 5 bis 60 % des Gesamtgewichts der Zusammensetzung ausmacht.

**22.** Zusammensetzung nach einem der vorhergehenden Ansprüche, **dadurch gekennzeichnet, dass** sie mindestens ein Farbmittel enthält.

23. Zusammensetzung nach dem vorhergehenden Anspruch, **dadurch gekennzeichnet, dass** das Farbmittel unter den in der Zusammensetzungen löslichen oder dispergierbaren Farbstoffen, Pigmenten, Perlglanzpigmenten und deren Gemischen ausgewählt ist.

24. Zusammensetzung nach Anspruch 22 oder 23, **dadurch gekennzeichnet, dass** das Farbmittel 0,5 bis 50 %, vorzugsweise 2 bis 40 % und besser 5 bis 30 %, bezogen auf das Gesamtgewicht der Zusammensetzung, ausmacht.

25. Zusammensetzung nach einem der vorhergehenden Ansprüche, **dadurch gekennzeichnet, dass** sie eine Partikelphase enthält.

26. Zusammensetzung nach einem der vorhergehenden Ansprüche, dass sie ferner mindestens ein Wachs enthält.

27. Zusammensetzung nach dem vorhergehenden Anspruch, **dadurch gekennzeichnet, dass** das Wachs 0,01 bis 50 %, vorzugsweise 2 bis 40 % und besser 5 bis 30 % des Gesamtgewichts der Zusammensetzung ausmacht.

28. Zusammensetzung nach einem der vorhergehenden Ansprüche, **dadurch gekennzeichnet, dass** sie ferner mindestens einen Zusatzstoff enthält, der unter den Antioxidantien, Konservierungsmitteln, Neutralisationsmitteln, lipophilen Gelbildnern oder nicht wässrigen flüssigen Verbindungen, Dispergiermitteln, kosmetischen Wirkstoffen und deren Gemischen ausgewählt ist.

29. Zusammensetzung nach einem der vorhergehenden Ansprüche, **dadurch gekennzeichnet, dass** sie mindestens einen kosmetischen Wirkstoff enthält, der unter den Vitaminen (A, E, C, $B_3$, F), Provitaminen, beruhigenden Stoffen, Pflanzenextrakten oder etherischen Ölen, Schutzmitteln oder restrukturierenden Wirkstoffen, erfrischenden Stoffen, Emollientien, Hydratisierungsmitteln, Antifaltenmitteln, essentiellen Fettsäuren und deren Gemischen ausgewählt ist.

30. Zusammensetzung nach einem der vorhergehenden Ansprüche, **dadurch gekennzeichnet, dass** sie als gegossenes oder kompaktiertes Produkt vorliegt.

31. Zusammensetzung nach einem der vorhergehenden Ansprüche, **dadurch gekennzeichnet, dass** sie als Lippenstift oder Lippenglanz vorliegt.

32. Kosmetisches Verfahren, um einem Film einer wasserfreien kosmetischen Zusammensetzung die Eigenschaften Farbbeständigkeit, Glanz, Komfort und/oder Nichtmigration zu geben, das darin besteht, in die Zusammensetzung mindestens eine nichtflüchtige Siliconverbindung, mindestens ein nicht siliconiertes, fettlösliches Polymer und mindestens ein Dispergiermittel auf Kohlenwasserstoffbasis einzubringen, das Solubilitätsparameter δd und δa aufweist, die die folgenden Bedingungen erfüllen: $16{,}2 \leq \delta_d \leq 20\ (J/cm^3)^{1/2}$ und $9{,}1 \leq \delta_a \leq 20\ (J/cm^3)^{1/2}$, wobei das Dispergiermittel Kohlenstoffatome und Wasserstoffatome und eine oder mehrere Funktionen aufweist, die unter den Funktionen Hydroxy, Ester, Ether, Carboxy und Amid ausgewählt sind, wobei die nichtflüchtige Siliconverbindung ein kosmetisches Öl ist, das Dispergiermittel auf Kohlenwasserstoffbasis 0,5 bis 40 % des Gesamtgewichts der Zusammensetzung ausmacht und die Zusammensetzung weniger als 10 % flüchtiges Öl, bezogen auf das Gesamtgewicht der Zusammensetzung, enthält.

33. Verfahren nach dem vorhergehenden Anspruch, **dadurch gekennzeichnet, dass** das Dispergiermittel auf Kohlenwasserstoffbasis ausgewählt ist unter:

- Ether-modifizierten Fettalkoholen und insbesondere Additionsprodukten von Ethylenoxid und/oder Propylenoxid mit i) einem geradkettigen oder verzweigten Fettalkohol oder ii) einem Alkylphenol,
- Estern, vorzugsweise Monoestern oder Diestern, die bei der Reaktion von Fettsäuren mit Additionsprodukten von Ethylenoxid und Glycerin oder Additionsprodukten von Ethylenoxid und Polyglycerin gebildet werden,
- Estern, die bei der Reaktion mindestens einer Fettsäure mit mindestens einem Additionsprodukt von Ethylenoxid und Glycerin oder mit mindestens einem Additionsprodukt von Ethylenoxid und Polyglycerin gebildet werden,
- Estern, die bei der Reaktion von Glycerin oder Polyglycerin mit mindestens einem Additionsprodukt von Ethylenoxid und einer gesättigten oder ungesättigten Fettsäure gebildet werden,
- partiellen Estern, die bei der Reaktion mindestens einer geradkettigen oder verzweigten, gesättigten oder ungesättigten Fettsäure, Ricinolsäure oder 12-Hydroxystearinsäure mit mindestens einem Polyol wie Glycerin, Polyglycerin, Pentaerythrit, Saccharidalkoholen wie Sorbit gebildet werden, und insbesondere Polyglycerylestern,

- Estern, die bei der Reaktion von Sorbitan mit mindestens einer geradkettigen oder verzweigten, gesättigten oder ungesättigten Fettsäure gebildet werden,
- Ether-modifizierten Sorbitanestern und insbesondere Estern, die iii) bei der Reaktion von Sorbitan mit mindestens einem Additionsprodukt von Ethylenoxid und einer gesättigten oder ungesättigten Fettsäure gebildet werden oder iv) die bei der Reaktion von mindestens einer gesättigten oder ungesättigten Fettsäure mit mindestens einem Additionsprodukt von Ethylenoxid und Sorbitan gebildet werden,
- Additionsprodukten von Ethylenoxid und Ricinusöl und/oder hydriertem Ricinusöl,
- Trialkylphosphaten und alkylierten Mono-, Di- und Triphosphaten, und deren Gemischen.

**34.** Verfahren nach Anspruch 32 oder 33, **dadurch gekennzeichnet, dass** die nichtflüchtige Siliconverbindung unter den nichtflüchtigen Polydimethylsiloxanen; Polydimethylsiloxanen, die als Seitenkette oder am Ende der Siliconkette Alkyl-, Alkoxy- oder Phenylgruppen aufweisen, wobei diese Gruppen 2 bis 24 Kohlenstoffatomen besitzen; Phenyltrimethiconen, Phenyldimethiconen, Phenyltrimethylsiloxydiphenylsiloxanen, Diphenyldimethiconen, Diphenylmethyldiphenyltrisiloxanen, 2-Phenylethyltrimethylsiloxysilicaten; fluorierten Siliconen, die als Seitenkette oder am Ende der Siliconkette eine fluorierte Gruppe mit 1 bis 12 Kohlenstoffatomen aufweisen, bei der alle oder ein Teil der Kohlenwasserstoffatome durch Fluoratome ersetzt sind; Siliconharzen; Silicongummis; und deren Gemischen ausgewählt ist.

**35.** Verfahren nach einem der Ansprüche 32 bis 34, **dadurch gekennzeichnet, dass** das nicht siliconierte, fettlösliche Polymer ausgewählt ist unter:

- verzweigten, nicht kristallinen Homopolymeren von Olefinen, nicht verzweigten Copolymeren von Olefinen, nicht kristallinen, hydrierten Homopolymeren und Copolymeren von Dienen, geradkettigen oder verzweigten, oligomeren Homo- oder Copolymeren von Alkyl(meth)acrylaten, die vorzugsweise eine $C_{8-30}$-Alkylgruppe enthalten und eine Molmasse von höchstens 10 000 g/mol besitzen, oligomeren Homo- und Copolymeren von perfluorierten Alkyl(meth)acrylaten, die eine Molmasse von höchstens 10 000 g/mol und noch besser 8000 g/mol oder darunter besitzen, oligomeren Homo- und Copolymeren von Vinylestern, die $C_{8-30}$-Alkylgruppen aufweisen und eine Molmasse von höchstens 10 000 g/mol und noch besser 8000 g/mol oder darunter besitzen, oligomeren Homo- und Copolymeren von Vinylethern, die $C_{8-30}$-Alkylgruppen aufweisen und eine Molmasse von höchstens 10 000 g/mol und noch besser 8000 g/mol oder darunter besitzen,
- fettlöslichen Polyethern, die bei der mehrfachen Veretherung eines oder mehrerer $C_{2-100}$-Diole und vorzugsweise $C_{2-50}$-Diole gebildet werden,
- nicht vernetzten Polyestern, die bei der Polykondensation einer geradkettigen oder verzweigten Dicarbonsäure oder Polycarbonsäure mit 4 bis 50 Kohlenstoffatomen und eines Diols oder Polyols mit 2 bis 50 Kohlenstoffatomen gebildet werden,
- und deren Gemischen.

**36.** Verwendung der Kombination aus mindestens einer nichtflüchtigen Siliconverbindung, mindestens eines nicht siliconierten, fettlöslichen Polymers und mindestens eines Dispergiermittels auf Kohlenwasserstoffbasis, das Solubilitätsparameter δd und δa aufweist, die die folgenden Bedingungen erfüllen: $16,2 \leq \delta_d \leq 20$ $(J/cm^3)^{1/2}$ und $9,1 \leq \delta_a \leq 20$ $(J/cm^3)^{1/2}$, wobei das Dispergiermittel Kohlenstoffatome und Wasserstoffatome und eine oder mehrere Funktionen aufweist, die unter den Funktionen Hydroxy, Ester, Ether, Carboxy und Amid ausgewählt sind, wobei die nichtflüchtige Siliconverbindung ein kosmetisches Öl ist, das Dispergiermittel auf Kohlenwasserstoffbasis 0,5 bis 40 % des Gesamtgewichts der Zusammensetzung ausmacht und wobei die Zusammensetzung weniger als 10 % flüchtiges Öl, bezogen auf das Gesamtgewicht der Zusammensetzung, enthält, in einer wasserfreien kosmetischen Zusammensetzung mit hoher Beständigkeit, insbesondere Farbbeständigkeit, die glänzt, angenehm ist und/oder nicht migriert.

**37.** Verwendung der Kombination aus mindestens einer nichtflüchtigen Siliconverbindung, mindestens eines nicht siliconierten, fettlöslichen Polymers und mindestens eines Dispergiermittels auf Kohlenwasserstoffbasis, das Solubilitätsparameter δd und δa aufweist, die die folgenden Bedingungen erfüllen: $16,2 \leq \delta_d \leq 20$ $(J/cm^3)^{1/2}$ und $9,1 \leq \delta_a \leq 20$ $(J/cm^3)^{1/2}$, wobei das Dispergiermittel Kohlenstoffatome und Wasserstoffatome und eine oder mehrere Funktionen aufweist, die unter den Funktionen Hydroxy, Ester, Ether, Carboxy und Amid ausgewählt sind, wobei die nichtflüchtige Siliconverbindung ein kosmetisches Öl ist, das Dispergiermittel auf Kohlenwasserstoffbasis 0,5 bis 40 % des Gesamtgewichts der Zusammensetzung ausmacht und die Zusammensetzung weniger als 10 % flüchtiges Öl, bezogen auf das Gesamtgewicht der Zusammensetzung, enthält, in einer wasserfreien kosmetischen Zusammensetzung als Stoff, um der Zusammensetzung die Eigenschaften Beständigkeit, insbesondere Farbbeständigkeit, Glanz, Komfort und/ oder Nichtmigration zu geben.

**38.** Verwendung nach Anspruch 36 oder 37, **dadurch gekennzeichnet, dass** das Dispergiermittel auf Kohlenwasserstoffbasis ausgewählt ist unter:

- Ether-modifizierten Fettalkoholen und insbesondere Additionsprodukten von Ethylenoxid und/oder Propylenoxid mit i) einem geradkettigen oder verzweigten Fettalkohol oder ii) einem Alkylphenol,
- Estern, die bei der Reaktion mindestens einer Fettsäure mit mindestens einem Additionsprodukt von Ethylenoxid und Glycerin oder mit mindestens einem Additionsprodukt von Ethylenoxid und Polyglycerin gebildet werden,
- Estern, die bei der Reaktion von Glycerin oder Polyglycerin mit mindestens einem Additionsprodukt von Ethylenoxid und einer gesättigten oder ungesättigten Fettsäure gebildet werden,
- partiellen Estern, die bei der Reaktion mindestens einer geradkettigen oder verzweigten, gesättigten oder ungesättigten Fettsäure, Ricinolsäure oder 12-Hydroxystearinsäure mit mindestens einem Polyol wie Glycerin, Polyglycerin, Pentaerythrit, Saccharidalkoholen wie Sorbit gebildet werden, und insbesondere Polyglycerylestern,
- Estern, die bei der Reaktion von Sorbitan mit mindestens einer geradkettigen oder verzweigten, gesättigten oder ungesättigten Fettsäure gebildet werden,
- Ether-modifizierten Sorbitanestern und insbesondere Estern, die iii) bei der Reaktion von Sorbitan mit mindestens einem Additionsprodukt von Ethylenoxid und einer gesättigten oder ungesättigten Fettsäure gebildet werden, oder iv) die bei der Reaktion von mindestens einer gesättigten oder ungesättigten Fettsäure mit mindestens einem Additionsprodukt von Ethylenoxid und Sorbitan gebildet werden,
- Additionsprodukten von Ethylenoxid und Ricinusöl und/oder hydriertem Ricinusöl,
- Trialkylphosphaten und alkylierten Mono-, Di- und Triphosphaten,

und deren Gemischen.

**39.** Verwendung nach einem der Ansprüche 36 bis 38, **dadurch gekennzeichnet, dass** die nichtflüchtige Siliconverbindung unter den nichtflüchtigen Polydimethylsiloxanen; Polydimethylsiloxanen, die als Seitenkette oder am Ende der Siliconkette Alkyl-, Alkoxy- oder Phenylgruppen aufweisen, wobei diese Gruppen 2 bis 24 Kohlenstoffatomen besitzen; Phenyltrimethiconen, Phenyldimethiconen, Phenyltrimethylsiloxydiphenylsiloxanen, Diphenyldimethiconen, Diphenylmethyldiphenyltrisiloxanen, 2-Phenylethyltrimethylsiloxysilicaten; fluorierten Siliconen, die als Seitenkette oder am Ende der Siliconkette eine fluorierte Gruppe mit 1 bis 12 Kohlenstoffatomen aufweisen, bei der alle oder ein Teil der Kohlenwasserstoffatome durch Fluoratome ersetzt sind; Siliconharzen; Silicongummis; und deren Gemischen ausgewählt ist.

**40.** Verwendung nach einem der Ansprüche 36 bis 40, **dadurch gekennzeichnet, dass** das nicht siliconierte fettlösliche Polymer ausgewählt ist unter:

- verzweigten, nicht kristallinen Homopolymeren von Olefinen, nicht verzweigten Copolymeren von Olefinen, nicht kristallinen, hydrierten Homopolymeren und Copolymeren von Dienen, geradkettigen oder verzweigten, oligomeren Homo- oder Copolymeren von Alkyl(meth)acrylaten, die vorzugsweise eine $C_{8-30}$-Alkylgruppe enthalten und eine Molmasse von höchstens 10 000 g/mol besitzen, oligomeren Homo- und Copolymeren von perfluorierten Alkyl(meth)acrylaten, die eine Molmasse von höchstens 10 000 g/mol und noch besser 8000 g/mol oder darunter besitzen, oligomeren Homo- und Copolymeren von Vinylestern, die $C_{8-30}$-Alkylgruppen aufweisen und eine Molmasse von höchstens 10 000 g/mol und noch besser 8000 g/mol oder darunter besitzen, oligomeren Homopolymeren und Copolymeren von Vinylethern, die $C_{8-30}$-Alkylgruppen aufweisen und eine Molmasse von höchstens 10 000 g/mol und noch besser 8000 g/mol oder darunter besitzen,
- fettlöslichen Polyethern, die bei der mehrfachen Veretherung eines oder mehrerer $C_{2-100}$-Diole und vorzugsweise $C_{2-50}$-Diole gebildet werden,
- nicht vernetzten Polyestern, die bei der Polykondensation einer geradkettigen oder verzweigten Dicarbonsäure oder Polycarbonsäure mit 4 bis 50 Kohlenstoffatomen und eines Diols oder Polyols mit 2 bis 50 Kohlenstoffatomen gebildet werden,
- und deren Gemischen.